(19) **Europäisches Patentamt / European Patent Office / Office européen des brevets**

(11) **EP 1 938 765 B1**

(12) **EUROPEAN PATENT SPECIFICATION**

(45) Date of publication and mention
of the grant of the patent:
**07.07.2010 Bulletin 2010/27**

(51) Int Cl.:
*A61B 17/56* (2006.01)    *A61B 17/58* (2006.01)
*A61B 17/72* (2006.01)    *A61F 2/46* (2006.01)
*A61M 25/10* (2006.01)

(21) Application number: **08075130.8**

(22) Date of filing: **01.06.1998**

(54) **Systems for treating fractured or diseased bone using expandable bodies**

Systeme zur Behandlung gebrochener oder erkrankter Knochen mithilfe dehnbarer Elemente

Système pour traiter un os fracturé ou malade à l'aide de corps expansibles

(84) Designated Contracting States:
**AT BE CH CY DE DK ES FI FR GB GR IE IT LI LU MC NL PT SE**

(30) Priority: **09.06.1997 US 871114**
            **15.08.1997 US 911805**
            **15.08.1997 US 911827**

(43) Date of publication of application:
**02.07.2008 Bulletin 2008/27**

(62) Document number(s) of the earlier application(s) in accordance with Art. 76 EPC:
**98925208.5 / 0 987 991**

(73) Proprietor: **Kyphon SÀRL**
**2000 Neuchâtel (CH)**

(72) Inventors:
• **Scholten, Arie**
**Fremont,**
**California 94536 (US)**

• **Reiley, Mark A**
**Piedmont,**
**California 94611 (US)**
• **Talmadge, Karen D**
**Los Altos**
**California 94022 (US)**
• **Scribner, Robert M.**
**Los Altos,**
**California 94024 (US)**

(74) Representative: **Desrousseaux, Grégoire Marie et al**
**Hirsch & Associés**
**58, Avenue Marceau**
**75008 Paris (FR)**

(56) References cited:
**EP-A- 0 621 020**    **EP-A- 1 426 075**
**WO-A-96/39970**    **US-A- 5 108 404**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The invention relates to the treatment of bone conditions in humans and other animals.

BACKGROUND OF THE INVENTION

When cancellous bone becomes diseased, for example, because of osteoporosis, avascular necrosis, or cancer, the surrounding cortical bone becomes more prone to compression fracture or collapse. This is because the cancellous bone no longer provides interior support for the surrounding cortical bone.

There are 2 million fractures each year in the United States, of which about 1.3 million are caused by osteoporosis alone.' There are also other bone disease involving infected bone, poorly healing bone, or bone fractured by severe trauma. These conditions, if not successfully treated, can result in deformities, chronic complications, and an overall adverse impact upon the quality of life.

U.S. Patents 4,969,888 and 5,108,404 disclose apparatus and methods for the fixation of fractures or other conditions of human and other animal bone systems, both osteoporotic and non-osteoporotic. The apparatus and methods employ an expandable body to compress cancellous bone and provide an interior cavity. The cavity receives a filling material, which hardens and provides renewed interior structural support for cortical bone.

Many interior regions of the body, such as the vasculature and interior bone, possess complex, asymmetric geometries. Even if an interior body region is somewhat more symmetric, it may still be difficult to gain access along the natural axis of symmetry.

For example, deployment of an expandable structure in the region of branched arteries or veins can place the axis of an expandable structure off-alignment with the axis of the blood vessel which the structure is intended to occupy. As another example, insertion of an expandable structure into bone can require forming an access portal that is not aligned with the natural symmetry of the bone. In these instances, expansion of the structure is not symmetric with respect to the natural axis of the region targeted for treatment. As a result, expansion of the body is not symmetric with respect to the natural' axis of the targeted region.

It can also be important to maximize the size and surface area of an expandable structure when deployed in an interior body region. Current medical balloons manufactured by molding techniques are designed to be guided into a narrow channel, such as a blood vessel or the fallopian tube, where they are then inflated. In this environment, the diameter of the balloon is critical to its success, but the length is less so. Such balloons only need to be long enough to cross the area of intended use, with few constraints past the effective portion of the inflated balloon. This allows conventional balloons to be constructed in three molded pieces, comprising a cylindrical middle section and two conical ends, bonded to a catheter shaft. As a practical matter, neither the length of the conical end, nor the length of the bond of the balloon to the catheter shaft, affect the function of conventional balloons, and these regions on conventional balloons are often 1 cm in length or more. Indeed, the larger the balloon diameter, the longer the end cone, which creates a tradeoff between maximum effective length and maximum effective diameter. This tradeoff makes optimization of conventional structures problematic in interior structures with defined lengths, such as bone.

US-5,108,404 discloses a method for fixing a vertebral body using an expandable member. The method involves forming a cavity in the vertebral body by introducing the expandable member into the interior of the body via a percutaneous access site provided by a cannula or guide pin.

EP-A-0621020 discloses multiple access channels provided by tubes. The first of these channels is used to insert a cavity forming gouge and to introduce elastic support members. The second channel is used to guide an observation instrument to the surgical site to monitor the clearing or implantation operations.

The better and more efficacious treatment of bone disease that these Patents promise can be more fully realized with improved systems for making and deploying expandable bodies in bone.

**Summary of the Invention**

According to the present invention there is provided a system for treating a vertebral body according to claim 1.

One embodiment of the invention provides systems for treating bone using an expendable wall in association with a nozzle for discharging a material. According to this embodiment, the systems insert both the body and the nozzle into a bone having cortical bone surrounding an interior volume occupied, at least in part, by cancellous bone. The systems cause the body to assume an expanded geometry while occupying the interior , volume in the presence of the nozzle to compact cancellous bone and form a cavity in the interior volume. The systems convey a material for discharge through the nozzle into the cavity at least partially while the body occupies the interior volume.

In a preferred embodiment, the systems convey bone cement for discharge through the nozzle, while the body is in the expanded geometry or a partially expanded geometry. The systems can also cause the expanded geometry of the body to decrease in volume in relation to volume of material discharged by the nozzle into the cavity.

In one embodiment, the expandable body and nozzle are deployed separately into the targeted bone. In a preferred embodiment, the expandable body and nozzle form a integrated tool and are deployed simultaneously into the targeted bone.

Another embodiment of the invention provides a device for deployment into an interior body region comprising a catheter tube, which carries an expandable structure. The structure is adapted to assume a collapsed geometry for deployment into the interior body region and an expanded geometry for use within the interior body region. The catheter tube extends along a first axis. The expanded geometry of the structure is oriented about a second axis, which is not aligned with the first axis. The asymmetry between the two axes permits deployment of the expandable structure in a symmetric fashion with respect to the natural axis of a targeted interior body region, even when the catheter tube is not aligned with the natural axis.

In a preferred embodiment, the device is intended to be inserted into bone having cortical bone surrounding an interior volume occupied, at least in part, by cancellous bone. In this embodiment, the expanded geometry of the structure compacts cancellous bone to form a cavity in the interior volume. The asymmetry between the catheter tube axis and the axis of the expanded structure geometry permits formation of a cavity central to the middle region of the interior volume, even when the access path the catheter tube follows does not align with any natural axis of the interior volume.

Another embodiment of the invention provides a device for deployment into an interior body region comprising a catheter tube which carries an expandable structure. The catheter tube has a first diameter, and the structure, when substantially expanded, has a second diameter greater than the first diameter. The structure includes spaced apart end regions. At least one of the end regions provides a non-conical diameter transition between the first and second diameters. The non-conical diameter transition makes it possible to achieve a desired maximum expanded diameter without an undesired reduction in the effective length of the structure.

This embodiment of the invention is advantageously used in the manufacture of devices intended to be inserted into cancellous bone to compact cancellous bone by expanding the structure and thereby form a cavity. The presence of non-conical end regions makes it possible to increase the volume of maximum cancellous bone compaction.

In a preferred embodiment, the ends regions are inverted about the catheter tube. The inverted end regions to make possible the compaction of cancellous bone along the entire length of the expandable structure.

Another embodiment of the invention provides a device for deployment in an interior body region comprising an inner catheter tube and an outer catheter tube. The distal end of the inner catheter extends beyond the distal end of the outer catheter. The proximal end of an expandable body is bonded to the distal end of the outer catheter tube, while the distal end of the expandable body is bonded to the distal end of the inner catheter tube. An expansion medium fills the interior of the expandable body through the space between the two catheter tubes. The inner catheter tube and the expandable body are made of materials which have more compliance than the outer catheter tube, so that the length of the expandable body grows proportionately with the diameter.

In a preferred embodiment, materials for the expandable body and inner catheter tube include more compliant polyurethanes, while materials for the outer catheter tube include less compliant polyurethanes or polyethylenes.

Another embodiment of the invention provides a device for deployment into an interior body region comprising a catheter tube, which carries an expandable structure and includes a stylet extending within the structure to stiffen it.

In a preferred embodiment, the stylet includes memory, which normally bends the stylet to deflect the structure relative to the catheter tube. The structure is slidably enclosed in a sheath during deployment. The sheath overcomes the memory of the stylet, to urge the stylet toward a generally straightened configuration. The memory bends the stylet and deflects the structure when the structure slides free of the sheath.

Another embodiment of the invention provides a sterile kit comprising a device for deployment in bone comprising a structure adapted to assume an expanded geometry having a desired configuration when used. The structure undergoes stress when expanded during its first use in bone. As a result, the structure can not be relied upon to reach its desired configuration during subsequent use in bone. The kit includes an overwrap defining a sealed sterile enclosure holding the device in a sterile condition until use. The kit verifies to the physician or user that the device packaged within it is sterile and has not been subjected to prior use. The physician or user is thereby assured that the expandable structure meets established performance and sterility specifications, and will have the desired configuration when expanded for use.

Features and advantages of the inventions are set forth in the following Description and Drawings, as well as in the appended Claims.

BRIEF DESCRIPTION OF THE DRAWINGS

Fig. 1 is a side view of the spinal column of a human;

Fig. 2 is coronal view of a lumbar vertebra, partially cut away and in section, taken generally along line 2-2 in Fig. 1;

Fig. 3 is a vertical section of lumbar vertebrae;

Fig. 4 is a plan view of a probe including a catheter tube carrying an expandable body intended to treat bone;

Figs. 5A to 5P are a series of coronal views of a vertebra, partially cut away and in section, showing the steps of introducing, via transpedicular access, an expandable body to compress cancellous bone and create a cavity within a vertebral body, and of then conveying a filling material into the cavity to restore interior integrity to cortical bone;

Fig. 5Q is a lateral view, with parts' broken away, of the vertebra shown in coronal view in Fig. 5P;

Fig. 6 is a coronal view of a vertebral body in which an expandable body, restrained by an external sealing element, compresses cancellous bone to form a cavity;

Fig. 7 is a coronal view, partially broken away and in section, of a vertebral body in which an expandable body is being collapsed after having formed a cavity, while an injector tip, also within the vertebral body, is simultaneously injecting filling material into the cavity;

Fig. 8A is a coronal view of a vertebral body, partially broken away and in section, showing a tool that integrates an injector tube and an integral expandable body to create a cavity in cancellous bone, and also shoving the injection of filling material simultaneous with collapse of the expandable body;

Fig. 8B is a side view of the tool shown in Fig. 8A, located outside bone;

Fig. 8C is sectional view of the tool shown in Fig. 8B, taken generally along line 8C-8C in Fig. 8B;

Fig. 9 is a coronal view of a vertebral body showing multiple expandable bodies separately introduced by transpedicular approach;

Fig. 10 is a view of: the distal end of a probe in which two catheter tubes, each carrying an expandable body, are joined to form a symmetric array, when substantially expanded outside a bone;

Fig. 11 is a view of the distal end of a probe in which two catheter tubes, each carrying an expandable body, are joined to form an asymmetric array, when substantially expanded outside a bone;

Fig. 12 is a coronal view, partially broken away and in section, of a vertebral body into which multiple expandable bodies have been deployed by dual transpedicular access;

Fig. 13 is a coronal view of a vertebral body, partially broken away and in section, into which multiple expandable bodies have been deployed by contralateral posterolateral access;

Fig. 14 is a coronal view of a vertebral body, partially broken away and in section, in which multiple expandable bodies have formed multiple cavities which join to form a single cavity to receive filling material;

Fig. 15 is a coronal view of a vertebral body, partially broken away and in section, in which multiple expandable bodies have formed multiple separate cavities to receive filling material;

Fig. 16 is an anterior-posterior view of a region of the spine, showing multiple expandable bodies present within a targeted vertebral body using ipsilateral postereolateral access;

Fig. 17 is an anterior-posterior view of a vertebral body, partially broken away and in section, in which multiple expandable bodies, introduced using ipsilateral postereolateral access, have formed multiple cavities which are joined to form a single cavity to receive filling material;

Fig. 18 is an anterior-posterior view of a vertebral body, partially broken away and in section, in which multiple expandable bodies, introduced using an ipsa posterolateral access, have formed multiple separate cavities to receive filling material;

Fig. 19 is a coronal view of a vertebral body, partially broken away and in section, in which multiple expandable bodies have been introduced by both transpedicular and posterolateral access;

Fig. 20 is a perspective view of one representative embodiment of an expandable body having a stacked doughnut-shaped geometry;

Fig. 21 is a view of another representative embodiment of an expandable body having an oblong-shaped geometry;

Fig. 22 is an elevation view of another representative embodiment of an expandable body showing three stacked bodies and string-like restraints for limiting the expansion of the bodies during inflation;

Fig. 23 is a perspective view of another representative embodiment of an expandable body having a kidney bean-shaped geometry;

Fig. 24 is a top view of another representative embodiment of an expandable body having a kidney bean-shaped geometry with several compartments by a heating element or branding tool;

Fig. 25 is a cross-sectional view taken along line 25-25 of Fig. 24;

Fig. 26 is a perspective, lateral view of a vertebral body, partially broken away to show the presence of an expandable body, and also' showing the major reference dimensions for the expandable body;

Fig. 27 is a dorsal view of an expandable body having a humpback banana-shaped geometry in use in a right distal radius;

Figs. 28 is a cross sectional view of the expandable body shown in Fig. 27, taken generally along line 28-28 of Fig. 27;

Fig. 29A is an expandable body having a spherical shape with a base, located in a proximal humerus and viewed from the front (anterior) of the left proximal humerus;

Fig. 29B is an expandable body having a cylindrical shape, located in a proximal humerus and viewed from the front (anterior) of the left proximal humerus;

Fig. 30A is an expandable body located, as shown in a front (anterior) view of the proximal tibia, introduced beneath the medial tibial plateau;

Fig. 30B is a side elevation view of the expandable body shown in Fig. 30A;

Fig. 30C is a top perspective view of the expandable body shown in Fig. 30A, showing its' generally cylindrical geometry;

Fig. 31 is a top plan view of another expandable body for use in treating tibial plateau fractures, having a generally elliptical geometry;

Fig. 32 is a side view of multiple expandable bodies stacked on atop another for use, for example, in treating tibial plateau fractures;

Fig. 33 is another expandable body having an egg-shaped geometry located, as shown in a front (anterior) view of the proximal tibia, introduced beneath the medial tibial plateau;

Fig. 34 is an expandable body having a spherical-shaped geometry for treating avascular necrosis of the head of the femur (or humerus), which is shown from the front (anterior) of the left hip;

Fig. 35 is a side view of another expandable body having a hemispherically-shaped geometry for treating avascular necrosis of the head of the femur (or humerus);

Fig. 36A is a view of an expandable body having a bent-geometry for preventing hip fracture, as seen from the front (anterior) of the left hip;

Fig. 36B is a view of multiple expandable bodies individually deployed through multiple access points into the left hip for preventing hip fracture;

Fig. 37A is a view of an expandable body having an asymmetric bow tie-shape for use in treating fracture of the calcaneus bone, shown in lateral view within the calcaneus;

Fig. 37B is a perspective top view of the expandable body shown in Fig. 37A when substantially expanded outside the calcaneus;

Fig. 38 shows an expandable body having a spherical or egg-shaped geometry shown in lateral view deployed within the calcaneus;

Figs. 39A to 39D show a multiple stage process of introducing filling material into a cavity formed by an expandable body in cancellous bone, to prevent or impede flow or seepage of filling material from the interior of the bone;

Fig. 40 is an elevation view of an injector tip for filling material, over which a mesh is draped, which, when deployed in a cavity formed by an expandable body, impedes or prevents seepage of the material from the cavity;

Fig. 41 is a coronal view of a vertebra, with parts broken away and in section, showing the deployment of the mesh shown in Fig. 40 within the vertebral body;

Figs. 42A to 42C are schematic illustrations of a method and system for delivering a therapeutic substance to a bone using an expandable body;

Fig. 43 is an illustration of the human skeleton, showing regions of long bone that can be treated using expandable bodies;

Fig. 44 shows multiple expandable bodies located, as shown in a front (anterior) view, within the proximal tibia, both introduced beneath the medial tibial plateau, one of the bodies being substantially expanded to form an interior barrier and serve as a platform for the other body, which is shown substantially collapsed;

Fig. 45 is a front (anterior) view of the multiple expandable bodies, shown in Fig. 44, with both bodies in substantially expanded conditions to form a cavity within the proximal tibia beneath the medial tibial plateau;

Fig. 46 is an enlarged front (anterior) perspective view of the multiple expandable bodies shown in Fig. 45, with the lower expandable body serving as a platform for the upper expandable body;

Fig. 47 is diagrammatic view of a system for harvesting bone marrow in a bone-marrow producing bone using an expandable body;

Fig. 48 is a section view of the catheter tube associated with the system shown in Fig. 48, taken generally along line 48-48 of Fig. 47;

Fig. 49 is an enlarged view of the expandable body associated with the system shown in Fig. 47 inside a bone for the purpose of harvesting bone marrow;

Fig. 50 is a top view of a probe including a catheter tube carrying a tubular expandable structure of conventional construction, shown in a substantially collapsed condition;

Fig. 52 is an enlarged side view of the tubular expandable structure carried by the probe shown in Fig. 50, shown in a substantially expanded condition;

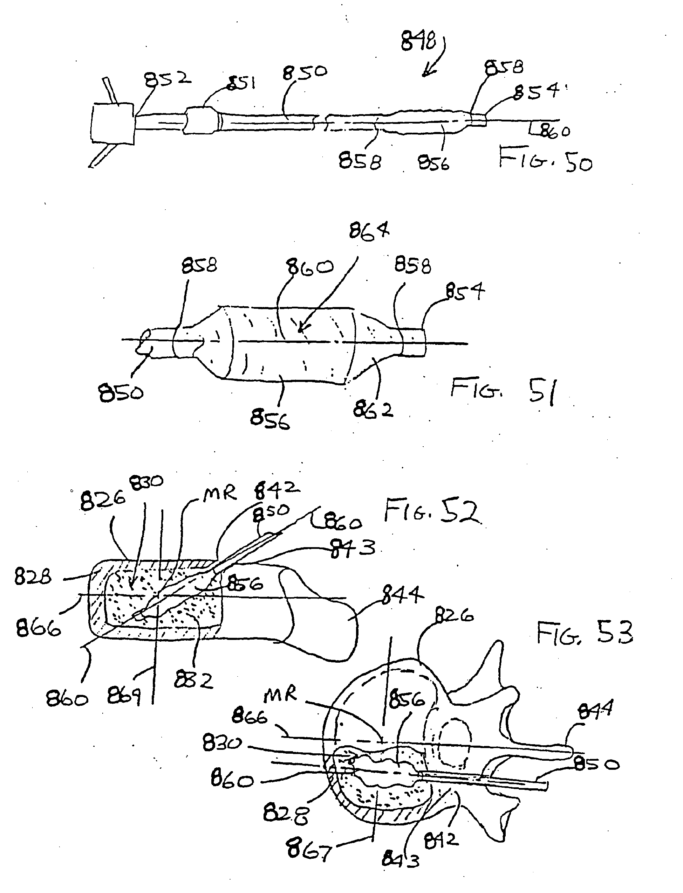

Fig. 52 is a lateral view of a lumbar vertebra, partially cut away and in section, with the expandable structure shown in Figs. 50 and 51 deployed when in a substantially collapsed condition;

Fig. 53 is a coronal view of the access shown in Fig. 52, partially cut away and in section;

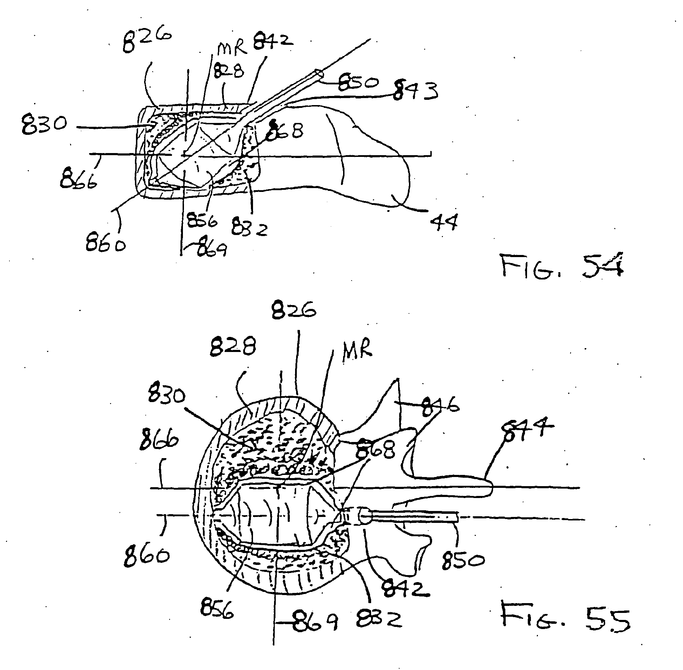

Fig. 54 is a lateral view of the access shown in Fig. 52 , with the expandable structure shown in Figs. 50 and 51 in a substantially expanded condition, forming a cavity that is not centered with respect to the middle region of the vertebral body;

Fig. 55 is a coronal view of the access shown in Fig. 54, partially cut away and in section;

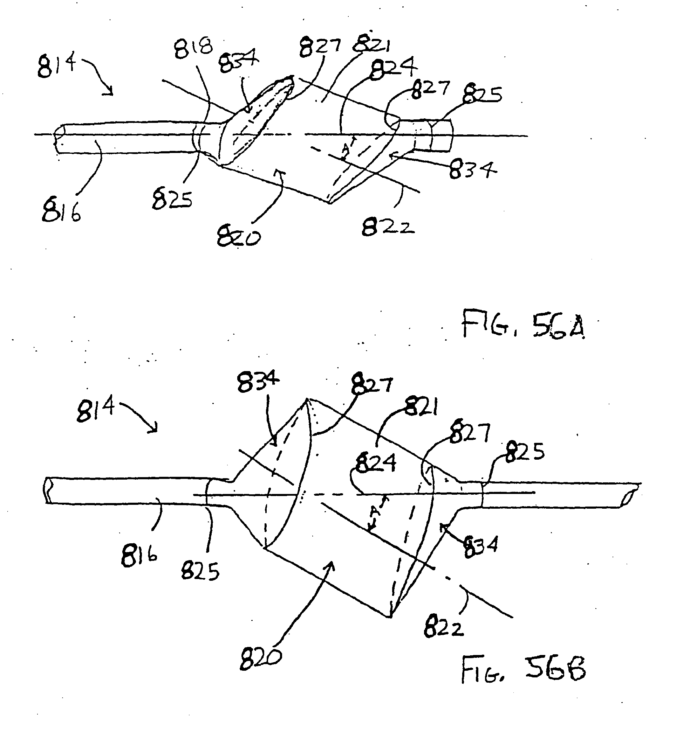

Figs. 56A and 56B are side views of improved expandable structures, each having an axis of expansion that is offset by an acute angle and not aligned with the axis of the supporting catheter tube;

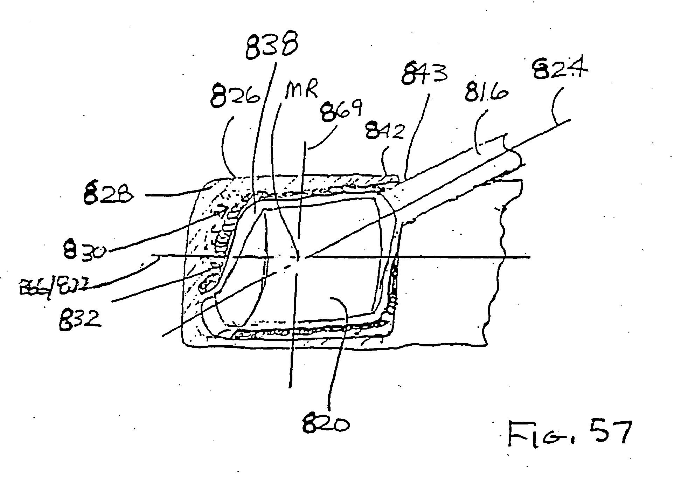

Fig. 57 is a lateral view of a lumbar vertebra, partially cut away and in section, with the offset expandable structure shown in Fig. 56A deployed and being in a substantially expanded condition, forming a cavity that is substantially centered with respect to the middle region of the vertebral body;

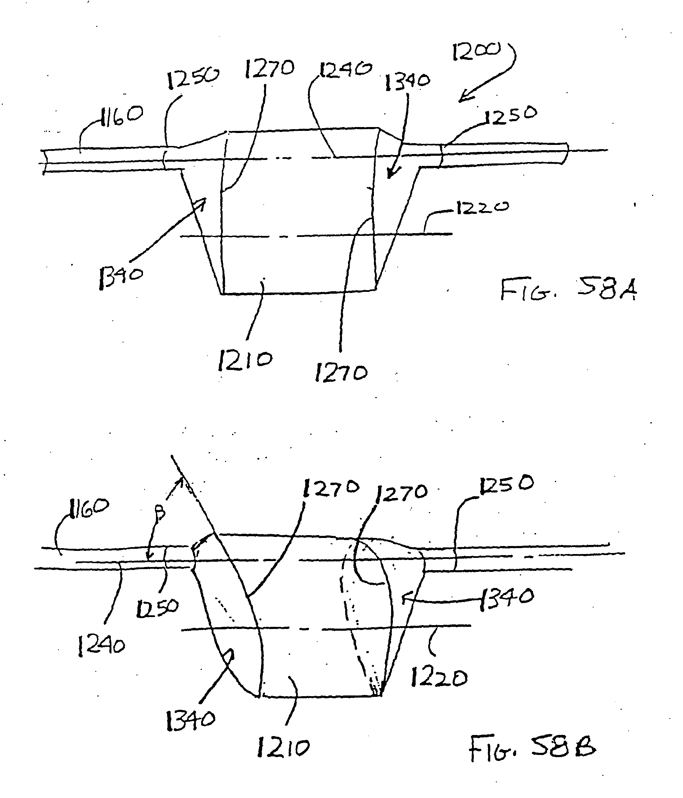

Figs. 58A and 58B are side views of other embodiments of improved expandable structures, each having an axis of expansion that is offset by a distance from the axis of the supporting catheter tube;

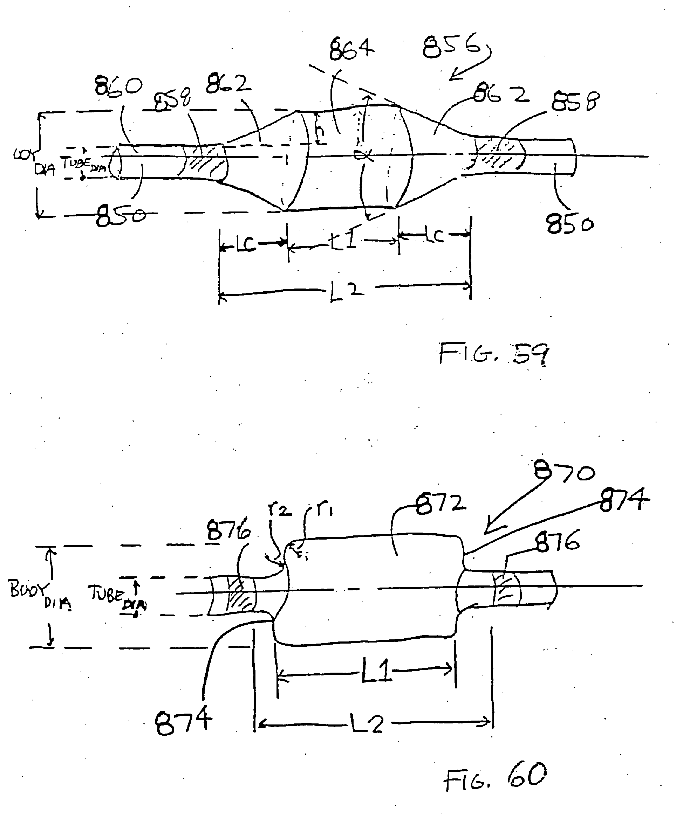

Fig. 59 is a side view of a conventional expandable structure shown in Fig. 51, enlarged to show further details of its geometry when substantially expanded;

Fig. 60 is a side view of an improved expandable structure, when in a substantially expanded condition, which includes end regions having compound curvatures that reduce the end region length and thereby provide the capability of maximum bone compaction substantially along the entire length of the structure;

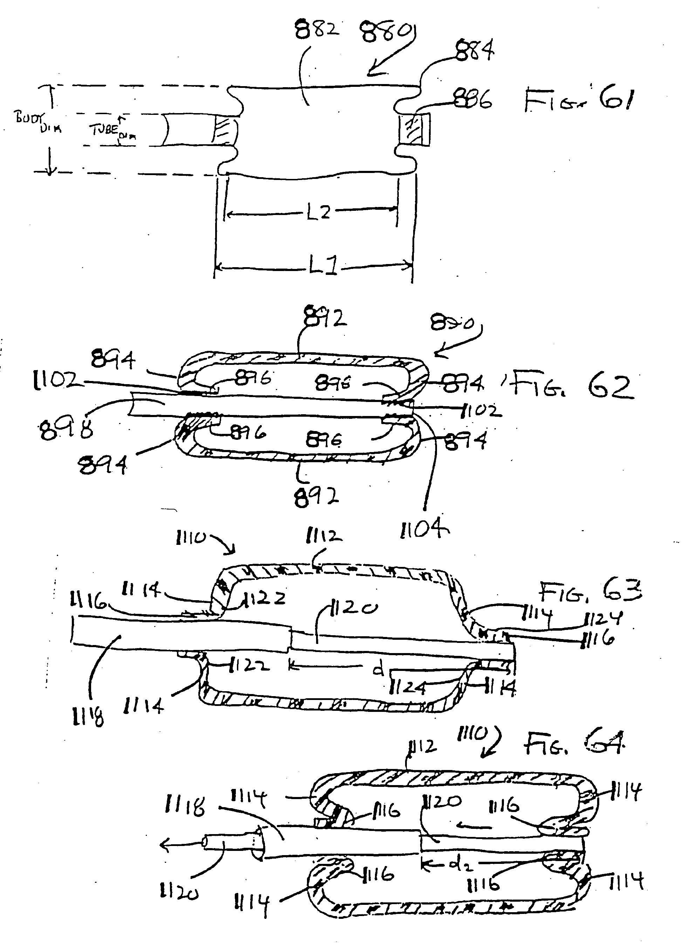

Fig. 61 is a side view of an improved expandable structure, when in a substantially expanded condition, which includes end regions having compound curvatures that invert the end regions about the terminal regions, where the structure is bonded to the supporting catheter tube, to provide the capability of maximum bone compaction substantially along the entire length of the structure;

Fig. 62 is a side section view of an improved expandable structure, when in a substantially expanded condition, which includes end regions that have been tucked or folded about the terminal regions, where the structure is bonded to the supporting catheter tube, to provide the capability of maximum bone compaction substantially along the entire length of the structure;

Fig. 63 is a side section view of a tubular expandable structure having a distal end bonded to an inner catheter tube and a proximal end bonded to an outer catheter tube, the inner catheter tube being slidable within the outer catheter tube;

Fig. 64 is a side section view of the tubular expandable structure shown in Fig. 63, after sliding the inner catheter tube within the outer catheter tube to invert the end regions of the structure about the distal and proximal bonds, to thereby provide the capability of maximum bone compaction substantially along the entire length of the structure;

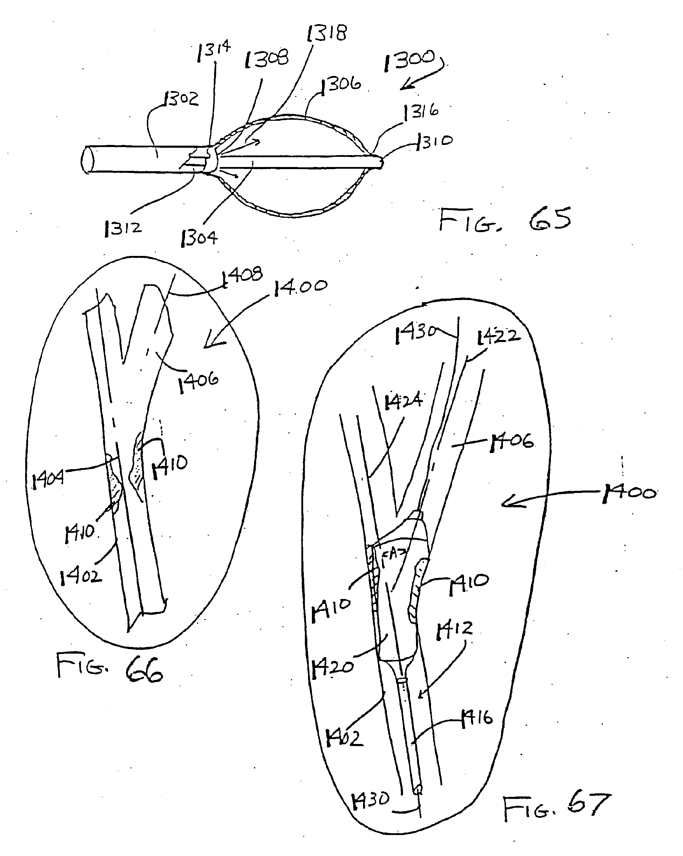

Fig. 65 is a side section view of a tubular expandable structure having a distal end bonded to an inner catheter tube and a proximal end bonded to an outer catheter tube, the inner catheter tube and structure being made of a more compliant material than the outer catheter tube to provide proportional length and diameter expansion characteristics;

Fig. 66 is an enlarged plan view of a branched blood vasculature region, in which an occlusion exists;

Fig. 67 is a further enlarged view of the branched blood vasculature region shown in Fig. 66, in which an asymmetric expandable structure of the type shown in Fig. 56A is deployed to open the occlusion;

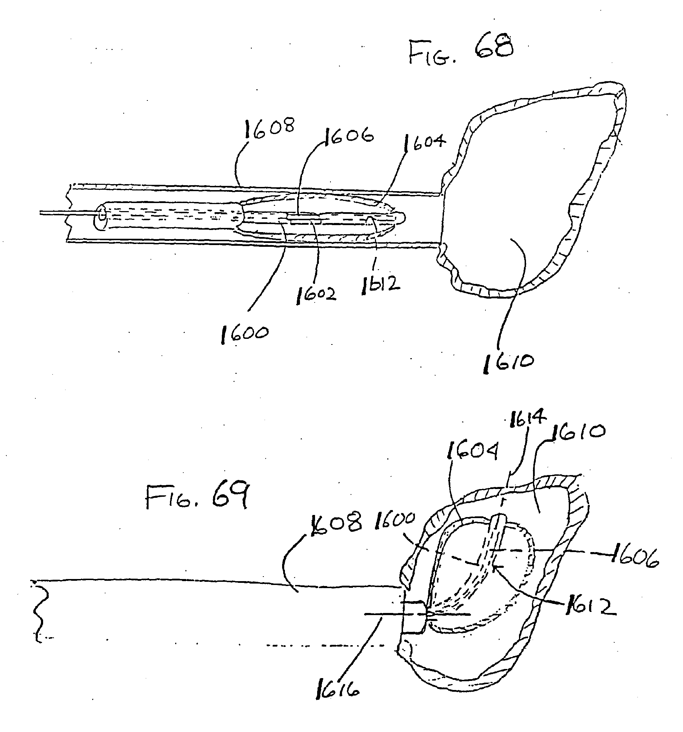

Fig. 68 is a side view, with parts broken away and in section, of an expandable structure having an enclosed stiffening member, to straighten the structure during passage through a guide sheath into an interior body region;

Fig. 69 is a side view of the expandable structure shown in Fig. 68, after deployment beyond the guide sheath and into the interior body region, in which the stiffening member includes a distal region having a preformed bend, which deflects the structure relative to the axis of the guide sheath;

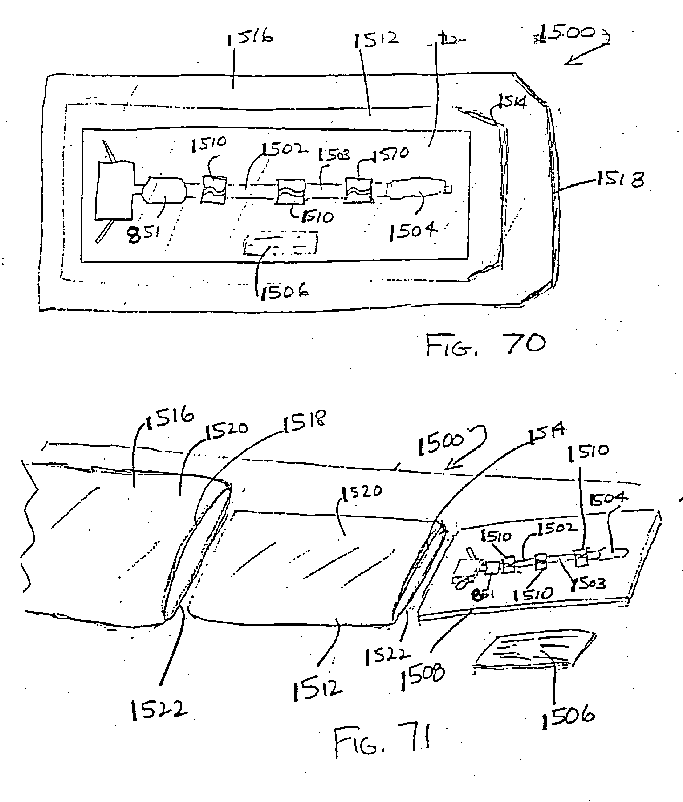

Fig. 70 is a plan view of a sterile kit to store a single use probe, which carries an expandable structures as previously shown; and

Fig. 71 is an exploded perspective view of the sterile kit shown in Fig. 70.

## DETAILED DESCRIPTION OF THE PREFERRED EMBODIMENTS

This Specification describes new systems and methods to treat bones using expandable bodies. The use of expandable bodies to treat bones is disclosed in United States Patent Numbers 4,969,888 and 5,108, 404 . Improvements in this regard are disclosed in United States Patent Application, Serial No. 08/188,224, filed January 26, 1994; United States Patent Application Serial Number 08/485,394, filed June 7, 1995; and United States Patent Application Serial Number 08/659,678, filed June 5, 1996.

The new systems and methods will be first described with regard to the treatment of vertebra. It should be appreciated, however, the systems and methods so described are not limited in their application to vertebrae. As will be described in greater detail later, the systems and methods are applicable to the treatment of diverse bone types.

**I. Treatment of Vertebral Bodies**

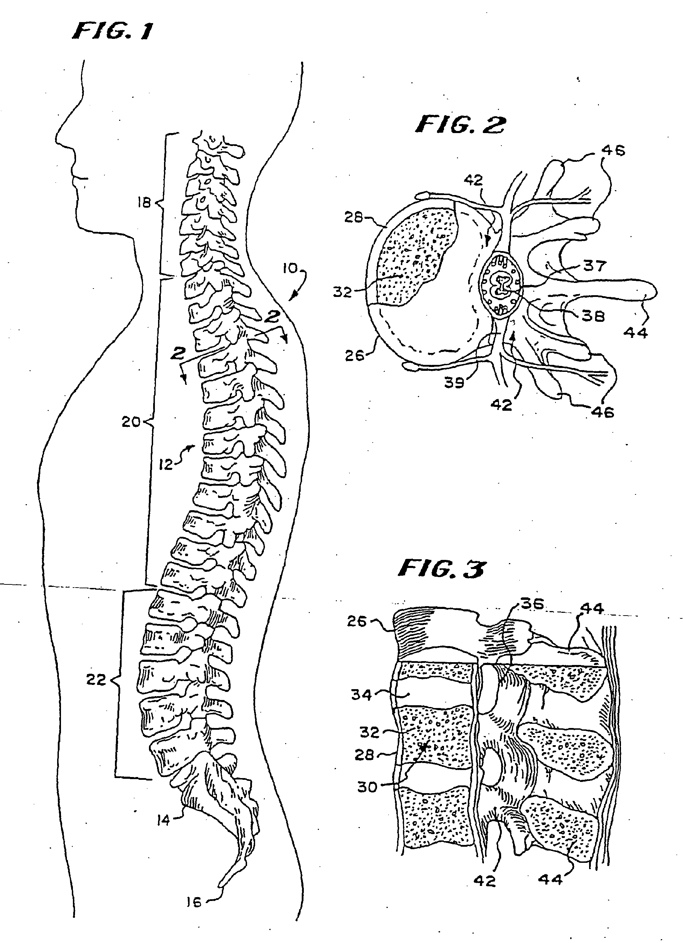

As Fig. 1 shows, the spinal column 10 comprises a number of uniquely shaped bones, called the vertebrae 12, a sacrum 14, and a coccyx 16(also called the tail bone) . The number of vertebrae 12 that make up the spinal column 10 depends upon the species of animal. In a human (which Fig. 1 shows), there are twenty-four vertebrae 12, comprising seven cervical vertebrae 18, twelve thoracic vertebrae 20, and five lumbar vertebrae 22.

When viewed from the side, as Fig. 1 shows, the spinal column 10 forms an S-shaped curve. The curve serves to support the head, which is heavy. In four-footed animals, the curve of the spine is simpler.

As Figs. 1 to 3 show, each vertebra 12 includes a vertebral body 26, which extends on the anterior (i.e., front or chest) side of the vertebra 12. As Figs. 1 to 3 show, the vertebral body 26 is in the shape of an oval disk. As Figs. 2 and 3 show, the vertebral body 26 includes an exterior formed from compact cortical bone 28. The cortical bone 28 encloses an interior volume 30 of reticulated cancellous, or spongy, bone 32 (also called medullary bone or trabecular bone). A "cushion," called an intervertebral disk 34, is located between the vertebral bodies 26.

An opening, called the vertebral foramen 36, is located on the posterior (i.e., back) side of each vertebra 12. The spinal ganglion 39 pass through the foramen 36. The spinal cord 38 passes through the spinal canal 37.

The vertebral arch 40 surrounds the spinal canal 37. The pedicle 42 of the vertebral arch 40 adjoins the vertebral body 26. The spinous process 44 extends from the posterior of the vertebral arch 40, as do the left and right transverse processes 46.

A. Deployment of an Expandable Body

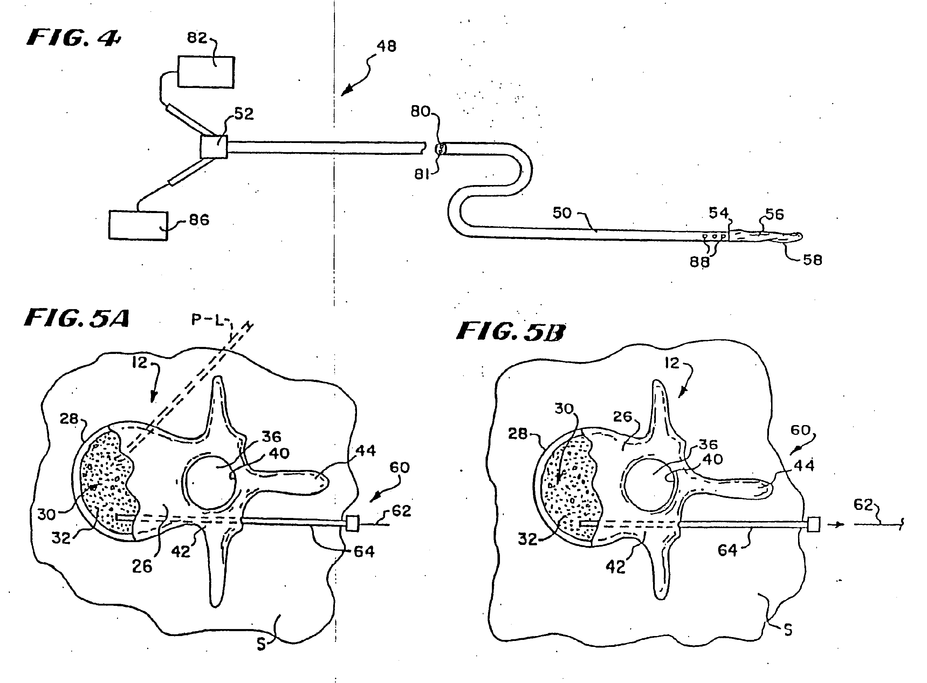

Fig. 4 shows a tool 48 for preventing or treating compression fracture or collapse of a vertebral body using an expandable body.

The tool 48 includes a catheter tube 50 having a proximal and a distal end, respectively 52 and 54. The distal end 54 carries an expandable body 56.

The body 56 includes an exterior wall 58, which, in Fig. 4, is shown in a collapsed geometry. The collapsed geometry permits insertion of the body 56 into the interior volume 30 of a targeted vertebral body 26.

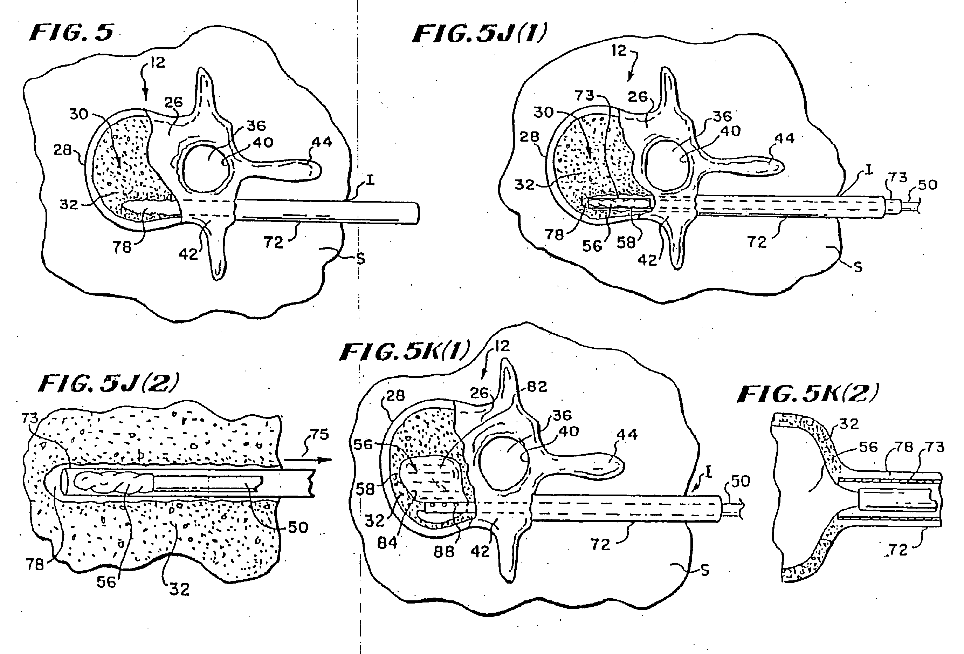

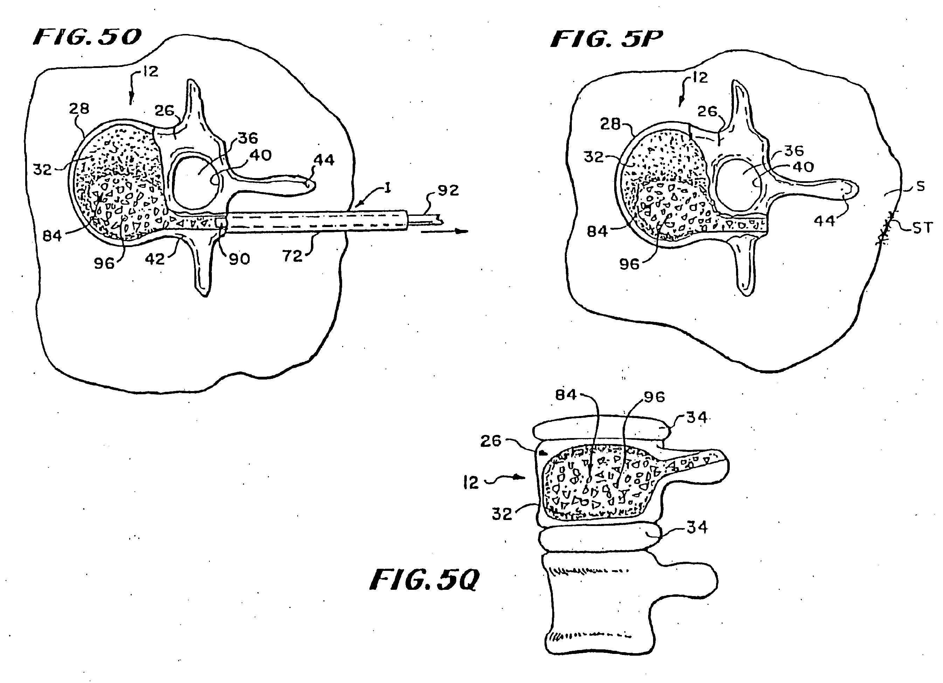

The insertion of the body 56 into the interior volume 30 of a targeted vertebral body 26 can be accomplished in various ways. Figs. 5A to 5Q show the insertion of the body 56 using a transpedicular approach, which can be performed either with a closed, mininimally invasive procedure or with an open procedure.

In the described procedure, a patient lies on an operating table, while the physician introduces a conventional spinal needle assembly 60 into soft tissue in the patient's back. The patient can lie facedown on the table, or on either side, or at an oblique angle, depending upon the physician's preference. Indeed, the procedure can be performed through an open anterior procedure or an endoscopic anterior procedure, in which case the tool 48 may be introduced from the anterior aspect of the vertebral body.

The spinal needle assembly 60 comprises a stylet 62 slidable housed within a stylus 64. The assembly 60 typically has, for example, about an 18 gauge diameter. Other gauge diameters can and will be used to accommodate appropriate guide pins, as will be described in greater detail later.

Under radiologic, CT, or MRI monitoring, the physician advances the assembly 60 through soft tissue (designated S in Fig. 5A) down to and into the targeted vertebra 12, as Fig. 5A shows. The physician will typically administer a local anesthetic, for example, lidocaine, through assembly 60. In some cases, the physician may prefer other forms of anesthesia.

The physician directs the spinal needle assembly 60 to penetrate the cancellous bone 32 of the targeted vertebra 12. Preferably the depth of penetration is about 60% to 95% of the vertebral body 26.

Fig. 5A shows gaining access to cancellous bone 32 through the pedicle 42, which is called transpedicular access. However, posterolateral access, through the side of the vertebral body 12 (designated P-L and shown in phantom lines in Fig. 5A), may be indicated, if a compression fracture has collapsed the vertebral body 26 below the plane of the pedicle 42, or for other reasons based upon the preference of the physician.

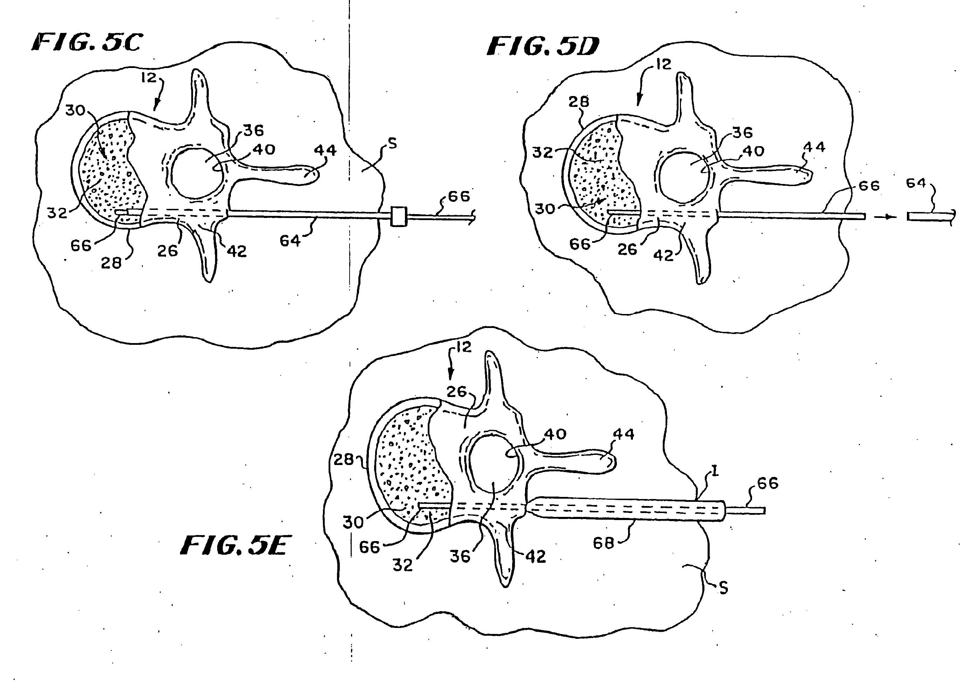

After positioning the spinal needle assembly 60 in cancellous bone 32, the physician holds the stylus 64 and withdraws the stylet 62 (see Fig. 5B). Still holding the stylus 64, the physician slides a guide pin 66 through the stylus 64 and into the cancellous bone 32(see Fig. 5C). The physician now removes the stylus 64, leaving the guide pin 66 deployed within the cancellous bone 32, as Fig. 5D shows.

As Fig. 5E shows, the physician makes a small incision (designated I in' Fig. 5E) in the patient's back to accommodate a trocar 68. The physician inserts the trocar 68 through the soft tissue S along the guide pin 66 down to the pedicle 42. The physician taps the distal end 70 of the trocar 68 into the pedicle 42 to secure its position.

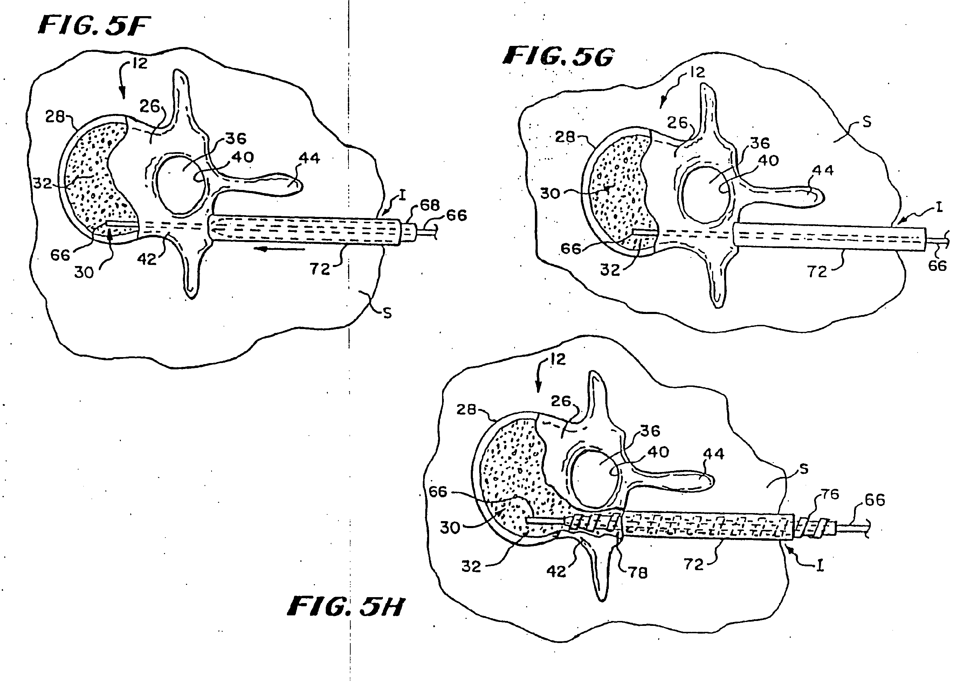

As Fig. 5F shows, the physician next slides an outer guide sheath 72 over the trocar 68. The distal end 74 of the outer guide sheath 72 is likewise tapped into the pedicle 42. The physician removes the trocar 68, leaving the guide pin 66 and outer guide sheath 72 in place, as Fig. 5G shows. Alternatively, the trocar 68 and guide sheath 72 can be introduced together in one step.

As Fig. 5H shows, the physician advances a drill bit 76 (for example, 5 mm in diameter) over the guide pin 66 through the outer guide sheath 72. Under X-ray control (or using another external visualizing system), the physician operates the drill bit 76 to open a passage 78 through the pedicle 42 and into the cancellous bone 32. The drilled passage 78 preferably extends no more than 95% across the vertebral body 26.

As Fig. 5I shows, the physician removes drill bit 76 and guide pin 66, leaving the outer guide sheath 72. The passage 78 made by the drill bit 76 remains, passing through the pedicle 42 and into the cancellous bone 32.

As Fig. 5J(1) shows, the physician next advances the catheter tube 50 and expandable body 56 through the outer guide sheath 72 and into the drilled passage 78 in the cancellous bone 32. As best shown in Fig. 5J(2), the body 56 is maintained in a straightened, collapsed condition distally beyond the end of the catheter tube 50 during transport through the guide sheath 72 and into the drilled passage 78 by a generally rigid, external protective sleeve 73, which surrounds the body 56. Alternatively, an internal stiffening member (not shown) can extend within the body 56, to keep the body 56 in the desired distally straightened condition during passage through the guide sheath 72. Once the body 56 is located in the desired location within the passage 78, the physician pulls the sleeve 73 back, to uncover the body 56. The expandable body 56 can be dipped into thrombin prior to its introduction into the vertebral body 26 to facilitate *in situ* coagulation.

The materials for the catheter tube 50 are selected to facilitate advancement of the body 56 into cancellous bone through the guide sheath 72. The catheter tube 50 can be constructed , for example, using standard flexible, medical grade plastic materials, like vinyl, nylon, polyethylenes, ionomer, polyurethane, and polyethylene tetraphthalate (PET). The catheter tube 50 can also include more rigid materials to impart greater stiffness and thereby aid in its manipulation. More rigid materials that can be used for this purpose include Kevlar™ material, PEBAX™ material, stainless steel, nickel-titanium alloys (Nitinol™ material), and other metal alloys.

Once the protective sheath 73 is withdrawn, the wall 58 of the body 56 is capable of assuming an expanded geometry within the interior volume 30 (generally shown in Fig. 5K(1)). To accommodate expansion of the body 56, the catheter tube 50 includes a first interior lumen 80 (see Fig. 4) . The lumen 80 is coupled at the proximal end of the catheter tube 50 to a pressurized source of fluid 82. The fluid 82 is preferably radio-opaque to facilitate visualization. For example, Renograffin™ can be used for this purpose.

The lumen 80 conveys the fluid 82 into the body 56 under pressure. As a result, the wall 58 expands, as Fig. 5K(1) shows. Because the fluid 82 is radio-opaque, body expansion can be monitored fluoroscopically or under CT visualization. Using real time MRI, the body 56 may be filled with sterile water, saline solution, or sugar solution.

Expansion of the wall 58 enlarges the body 56 and compacts cancellous bone 32 within the interior volume 30. As Fig. 5K(2) shows, the presence of the sheath 73 serves to keep the proximal end of the body 56 away from edge-contact with the distal end of the catheter tube 50.

The compaction of cancellous bone 32 forms a cavity 84 in the interior volume 30 of the vertebral body 26. The compaction of cancellous bone also exerts interior force upon cortical bone, making it possible to elevate or push broken and compressed bone back to or near its original prefracture position. Using a single transpedicular access (as Fig. 5K(1) shows), the cavity 84 occupies about one-half of the interior volume 30. As will be described in greater detail later, using multiple accesses, e.g., one through each pedicle, a cavity 84 occupying substantially all of the interior volume 30 can be created.

As Fig. 4 shows, the proximal end of the catheter tube 50 is preferably coupled by tubing to a source of negative air pressure 86. The negative pressure is conveyed through a second interior lumen 81 to one or more suction holes 88 on the distal end of the catheter tube 50. Prior to and during the expansion of the body 56, suction is applied to remove fats and other debris through the suction holes 88 for disposal. A separate suction-irrigation tool can be deployed through the guide sheath 72 for this purpose, if desired.

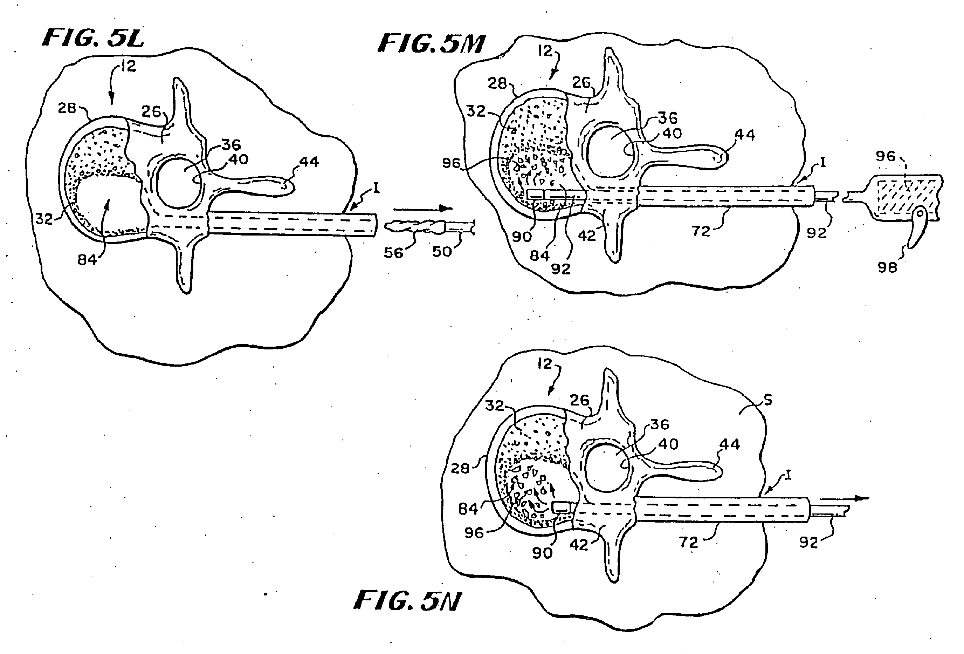

The body 56 is preferably left inflated for an appropriate waiting period, for example, three to five minutes, to allow coagulation inside the vertebral body 26. After the appropriate waiting period, the physician collapses the body 56 and removes it through the outer guide sheath 72 (see Fig. 5L). To facilitate removal, the exterior surface of the body 56 can be treated, e.g., by ion beam-based surface treatment, to reduce friction during passage through the outer guide sheath 72. As Fig. 5L shows, upon removal of the body 56, the formed cavity 84 remains in the interior volume 30.

A suction-irrigation tool (not shown) can be introduced through the outer guide sheath 72, to further flush and clear debris from the formed cavity 84 after removal of the body 56.

As Fig. 5M shows, an injector nozzle or tip 90, coupled by an injector tube 92 to an injector gun 94, is inserted through the outer guide sheath 72 into the formed cavity 84. The injector gun 94 carries a filling material 96. The filling material 96 comprises, for example, methylmethacrylate cement or a synthetic bone substitute.

The injector gun 94 can comprise a cement gun made, for example, by Stryker Corporation (Kalamazoo, Michigan). This particular injector gun 94 has a manually operated injection grip 98 with a mechanical advantage of about 9 to 1. Other injection guns may be used, having more or less mechanical advantage. Non-manually operated injection guns can also be used.

The injector tip 90 can be, for example, about 4.9 mm in diameter, to accommodate the flow a relatively viscous material 96 into the cavity 84.

As Fig. 5M shows, the injector gun 94 pushes the filling material 96 into the cavity 84. While injecting the material 96, the physician preferably begins with the injector tip 90 positioned at the anterior region of the cavity 84 (as Fig. 5M shows) . The physician progressively moves the tip 90 toward the posterior region of the cavity 84 (as Fig. 5N shows), away from the flow of the material 96 as it enters and fills the cavity 84. The physician observes the progress of the injection fluoroscopically.

The physician can also check, using, for example, x-ray, for leakage of the material through cortical bone 28. Systems and methods for impeding or preventing such leakage will be described in greater detail later.

The physician flows material 96 into the cavity 84, until the material 96 reaches the distal end 74 of the outer guide sheath 72 (as Fig. 50 shows).

Upon removing the injector tube 92 from the outer guide sheath 72, the physician may, if necessary, tamp residual filling material 96 from the distal end 74 of the outer guide sheath 72 into the cavity 84. If fluoroscopic examination reveals void regions in the cavity 84, the physician may again insert the injector tube 92 to add more filling material 96 into the cavity 84.

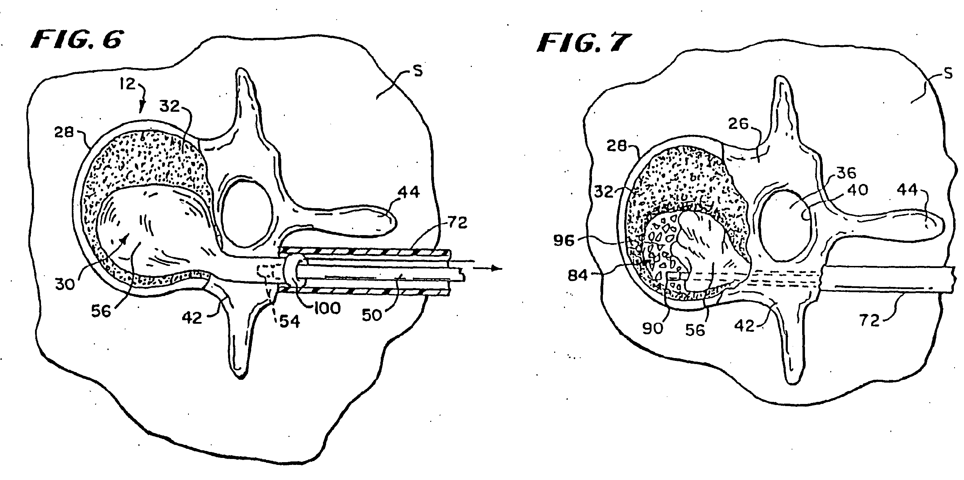

Fig. 7 shows an alternative technique for filling the cavity. In this technique, the injector tip 90 occupies the cavity 84 while the expandable body 56 is collapsing within the cavity 84. As the body 56 collapses, the tip 90 injects material 96 into the part of the cavity 84 that the collapsing body 56 no longer occupies. The increasing volume of the cavity 84 not occupied by the collapsing body 56 is thereby progressively filled by an increasing volume of material 96. The presence of the body 56, partially expanded while the tip 90 injects the material 96, serves to compact and spread the injected material 96 within the cavity 84.

As filling of the cavity 84 progresses, preferably under fluoroscopic monitoring, the physician progressively retracts the injector, tip 90 from the anterior region of the cavity 84, toward the outer guide sheath 72, allowing the material 96 to progressively enter and fill the cavity 84 with the collapse of the body 56.

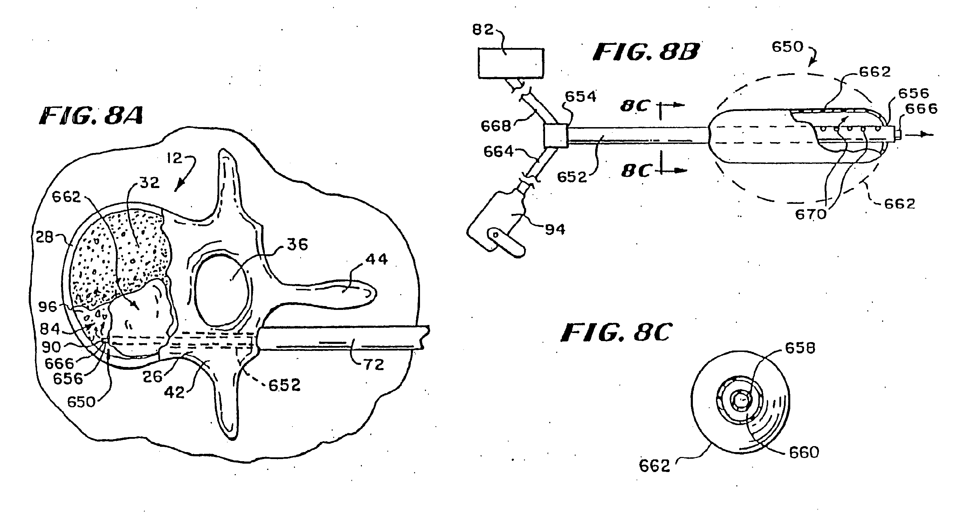

Figs. 8A to 8C show a preferred embodiment of a tool 650 which integrates the injection tube and expandable body in a single structure. As Fig. 8B shows, the tool 650 includes a catheter tube 652 having a proximal end 654 and a distal end 656. The distal end carries an expandable body 662.

As Fig. 8C shows, the catheter tube 652 has concentric inner and outer lumens, respectively 658 and 660. The inner lumen 658 communicates, by proximal tubing 664, with an injector gun 94, of the type previously described. The inner lumen 658 also communicates with an injector nozzle or tip 666 at the distal catheter tube end 656. Operation of the gun 94 serves to inject filling material 96 through the nozzle 666 (as Fig. 8A shows).

The outer lumen 660 communicates, via proximal tubing 668, with a source 82 of pressurized liquid. The outer lumen 660 also communicates with ports 670 formed on the distal catheter tube end 656 underlying the expandable body 662. Operation of the source 82 serves to inject pressurized liquid into the body 662 to expand it, in the manner previously described.

As Fig. 8A shows, the physician introduces the tool 650 into the cancellous bone 32. The physician expands the body 662 to create the cavity 84. Once the cavity 84 is formed, the physician begins to collapse the body 662, while injecting the filling material 96 through the nozzle 666. The volume of the cavity 84 occupied by the collapsing body 662 is progressively filled by the increasing volume of filling material 96 injected through the nozzle 666.

As earlier described, the collapsing body 662 serves to compact and spread the filling material 96 more uniformly within the cavity 84. Under fluoroscopic monitoring, the physician progressively retracts the distal end 656 of the tool 650 from the anterior region of the cavity 84 toward the outer guide sheath 72, allowing the material 96 to enter and fill the cavity 84.

Upon filling the cavity 84 with the material 96, the physician removes the outer guide sheath 72, as Figs. 5P and 5Q show. The incision site is sutured or otherwise closed (designated by ST in Fig. 5P).

In time, the filling material 96 sets to a hardened condition within the cavity 84 (see Figs. 5P and 5Q). The hardened material 96 provides renewed interior structural support for the cortical bone 28.

The above described procedure, carried out in a minimally invasive manner, can also be carried out using an open surgical procedure. Using open surgery, the physician can approach the bone to be treated as if the procedure is percutaneous, except that there is no skin and other tissues between the surgeon and the bone being treated. This keeps the cortical bone as intact as possible, and can provide more freedom in accessing the interior volume 30 of the vertebral body.

## B. Material Selection for the Expandable Body

The material of the body wall 58 can be selected according to the therapeutic objectives surrounding its use. For example, materials including vinyl, nylon, polyethylenes, ionomer, polyurethane, and polyethylene tetraphthalate (PET) can be used. The thickness of the body wall 58 is typically in the range of 0.051 to 0.645 mm (2/1000ths to 25/1000ths of an inch), or other thicknesses that can withstand pressures of up to, for example, 1723 to 3447 kPa (250-500 psi).

If desired, the material for the wall 58 can be selected to exhibit generally elastic properties, like latex. Alternatively, the material can be selected to exhibit less elastic properties, like silicone. Using expandable bodies 56 with generally elastic or generally semi-elastic properties, the physician monitors the expansion to assure that over-expansion and wall failure do not occur. Furthermore, expandable bodies 56 with generally elastic or generally semi-elastic properties will require some form of external or internal restraints to assure proper deployment in bone.

For example, expandable bodies 56 with generally elastic properties will exhibit the tendency to backflow or creep into the outer guide sheath 72 during their expansion. It is therefore necessary to internally or externally restrain a body 56 that is subject to creeping, to keep it confined within the interior bone region. In Fig. 6, an exterior sealing element 100 is provided for this purpose. In Fig. 6, the sealing element 100 takes the form of a movable o-ring.

The physician advances the o-ring 100 along the catheter tube 50 inside the guide sheath 72 using a generally stiff stylet 102 attached to the o-ring 100. The physician locates the o-ring 100 at or near the distal end 54 of the catheter tube 50 prior to conveying the liquid 82 to expand the body 56. The o-ring 100 is held in place by the generally stiff stylet 102, which provides a counter force to prevent backward movement of the o-ring 100 in the guide sheath 72 as the body 56 expands. The o-ring 100 thereby keeps all or a substantial portion of the generally elastic body 26 confined inside the interior volume 30. The body 56 thereby serves to compact as much of the cancellous bone 32 as possible.

The use of an external sealing element 100 to restrain the expandable body 56 may not be necessary when relatively inelastic materials are selected for the body 56. For example, the material for the body wall 58 can be selected to exhibit more inelastic properties, to limit expansion of the wall 58 prior to wall failure. The body wall 58 can also include one or more restraining materials, particularly when the body wall 58 is itself made from more elastic materials. The restraints, made from flexible, inelastic high tensile strength materials, limit expansion of the body wall 58 prior to wall failure. Representative examples of generally inelastic wall structures will be described in greater detail later.

## C. Selection of shape and size for the Expandable Body

As will also be demonstrated later, when relatively inelastic materials are used for the body wall 58, or when the body wall 58 is otherwise externally restrained to limit its expansion prior to failure, a predetermined shape and size can be imparted to the body 56, when it is substantially expanded. The shape and size can be predetermined according to the shape and size of the surrounding cortical bone 28 and adjacent internal structures, or by the size and shape of the cavity 84 desired to be formed in the cancellous bone 32.

In one embodiment, which is generally applicable for treating bones experiencing or prone to fracture, the shape and size of the body 56, when substantially expanded, can be designed to occupy at least about 30% of the volume of cancellous bone 32 in the interior volume 30. A body 56 having a substantially expanded size and shape in the range of about 40% to about 99% of the cancellous bone volume is preferred.

In another embodiment, which is applicable for treating bones having more localized regions of fracture or collapse caused, for example, by avascular necrosis, the shape and size of the body 56 can be designed to occupy as little as about 10% of the cancellous bone volume. In this embodiment, the drilled passage 78 extends directly to the localized site of injury, to enable targeted introduction of the body 26.

The shape of the cancellous bone 32 to be compressed, and the presence of surrounding local anatomic structures that could be harmed if cortical bone were moved inappropriately, are generally understood by medical professionals using textbooks of human skeletal anatomy, along with their knowledge of the site and its disease or injury. The physician is also able to select the materials and geometry desired for the body 56 based upon prior analysis of the morphology of the targeted bone using, for example, plain films, spinous process percussion, or MRI or CRT scanning. The materials and geometry of the body 56 are selected to create a cavity 84 of desired size and shape in cancellous bone 32 without applying harmful pressure to the outer cortical bone 28 or surrounding anatomic structures.

In some instances, it is desirable, when creating the cavity 84, to move or displace the cortical bone 28 to achieve the desired therapeutic result. Such movement is not *per se* harmful, as that term is used in this Specification, because it is indicated to achieve the desired therapeutic result. By definition, harm results when expansion of the body 56 results in a worsening of the overall condition of the bone and surrounding anatomic structures, for example, by injury to surrounding tissue or causing a permanent adverse change in bone biomechanics.

## D. Deployment of Multiple Expandable Bodies

Formation of a desired cavity geometry in cancellous bone 32 using an expandable body 56 can be accomplished in diverse ways to achieve the desired therapeutic effect. The foregoing disclosure envisions the deployment of a single expandable body 56 to compact cancellous bone 32 and, by itself, form a cavity 84 having a desired shape and size to receive a filling material 96.

Alternatively, a cavity 84 having a desired shape and size in cancellous bone 32 can be formed by the deployment of more than one expandable body 56 in a targeted region of cancellous bone 32, either sequentially or simultaneously.

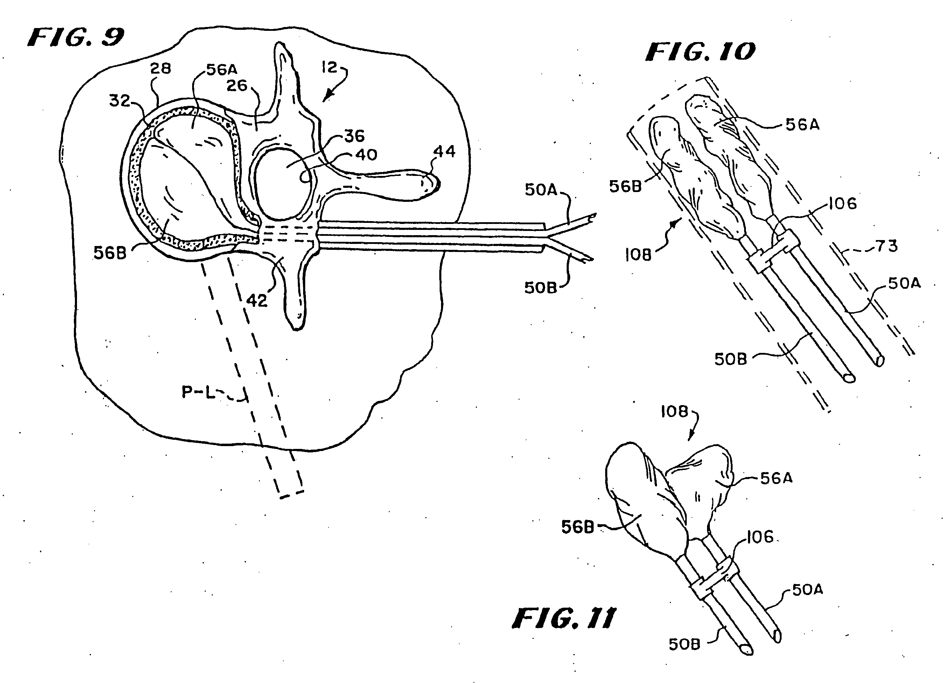

Fig. 9 shows the representative deployment of multiple expandable bodies 56A and 56B through a single outer guide sheath 72, which is arranged to provide transpedicular access. It should be understood that deployment of multiple expandable bodies can likewise be achieved through an outer guide sheath 72 arranged to provide a posterolateral access, through the side of the vertebral body 26 (as shown as P-L in phantom lines in Fig. 9). In Fig. 9, the expandable bodies 56A and 56B' are carried by separate catheter tubes 50A and 50B, which are not joined together.

In the alternative embodiment shown in Fig. 10, a tool 109 comprising an array 108 of catheter tubes 50A and 50B is provided. Each catheter tube 50A and 50B each carries an expandable body 56A and 56B, which are shown in Fig. 10 in a collapsed condition. In Fig. 10, the distal ends of the catheter tubes 50A and 50B are joined by a connector 106, for simultaneous deployment through an outer guide sheath 72 into the vertebral body 26, as Fig. 9 shows. As before described, a slidable protective sheath 73 encloses the bodies 56A and 56B during passage through the guide sheath 72. Upon withdrawal of the protective sheath 73, expansion of the bodies 56A and 56B, either simultaneously or sequentially, creates a cavity 84. If desired, the connector 106 can permit relative adjustment of the catheter tubes 50A and 50B, so that, when deployed, one expandable body is located more distal to another expandable body.

For the sake of illustration, Figs. 9 and 10 show two catheter tubes 50A and 50B, but more than two catheter tubes can be deployed in the vertebral body 26, either as separate tools (as Fig. 9 shows), or joined to form a composite array 108 (as Fig. 10 shows).

In Fig. 10, the bodies 56A and 56B of the array 108 have generally the same geometry, when substantially expanded, thereby providing a symmetric arrangement for compacting cancellous bone 32. A generally symmetric cavity 84 results.

Alternatively, as shown in Fig. 11, the bodies 56A and 56B possess different geometries when substantially expanded, thereby presenting an asymmetric arrangement for compacting cancellous bone 32. A generally asymmetric cavity 84 results. By mutually adjusting catheter tubes through a connector 106 (as previously described), the distal extensions of expandable bodies relative to each other can be made to differ, thereby also resulting in asymmetric cavity formation.

The selection of size and shape of the array 108, whether symmetric or asymmetric, depends upon the size and shape of the targeted cortical bone 28 and adjacent internal structures, or by the size and shape of the cavity 84 desired to be formed in the cancellous bone 32. The deployment of multiple expandable bodies 56 makes it possible to form cavities 84 having diverse and complex geometries within bones of all types. Multiple expandable bodies having generally the same geometry can be deployed in different ways to create cavities of different geometries.

It should be appreciated that the various styles of multiple expandable bodies 56 shown in Figs. 9 to 11 are deployed in a distally straightened condition (as Figs. 10 and 11 show) by using, e.g., a relatively stiff, surrounding sheath 73 (shown in phantom lines in Fig. 10), which is manipulated in the same as previously described in connection wi th Figs. 5J(1) and 5J(2). There are, of course, other ways to straighten the bodies 56 for deployment into bone, such as through the use of internal stiffening elements.

Access for expandable bodies 56 can be achieved through multiple access sites and in many different ways. For example, multiple expandable bodies can access the vertebral body from different regions of a targeted vertebra.

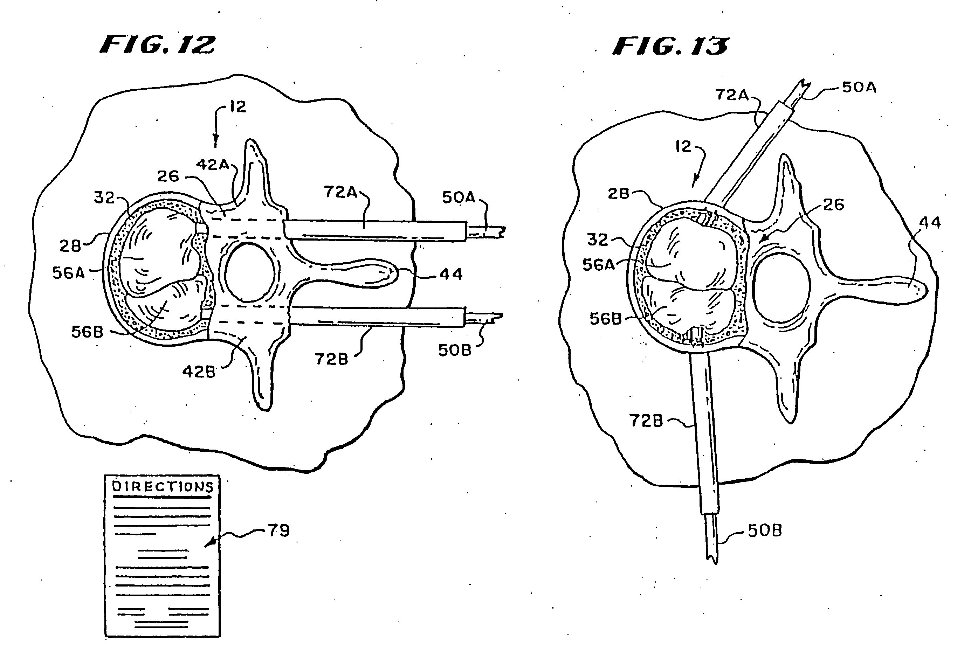

Fig. 12 shows a representative dual transpedicular access, in which two outer guide sheaths 72A and 72B are used to provide separate access for two or more expandable bodies 56A and 56B through different sides of the pedicle 42A and 42B of the vertebral body 26.

Fig. 13 shows a representative dual contra lateral posterolateral access, in which two outer guide sheaths 72A and 72B are used to provide separate access for multiple expandable bodies 56A and 56B from different lateral sides of the vertebral body 26.

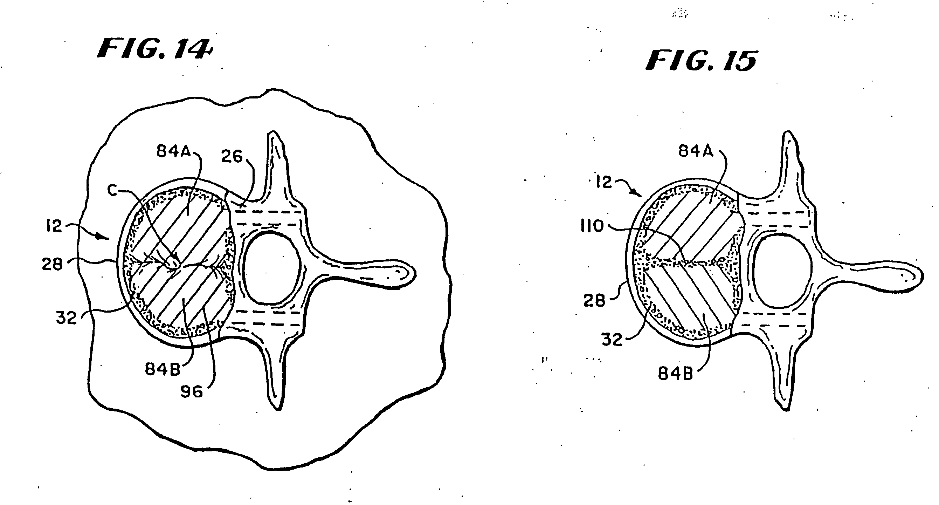

Deployed from dual access sites as shown in Figs. 12 and 13, the multiple expandable bodies 56A and 56B each forms a cavity 84A and 84B (shown in Fig. 14). The cavities 84A and 84B are transversely spaced within the cancellous bone 32. The transversely spaced cavities 84A and 84B may adjoin to form a single combined cavity (designated C in Fig. 14), into which the filling material 96 is injected. Alternatively, as Fig. 15 shows, the transversely spaced cavities 84A and 84B may remain separated by a region of cancellous bone (designated by numeral 110 in Fig. 13) . In this arrangement, the filling material 96 is injected into multiple, individual cavities 84A and 84B within the interior volume.

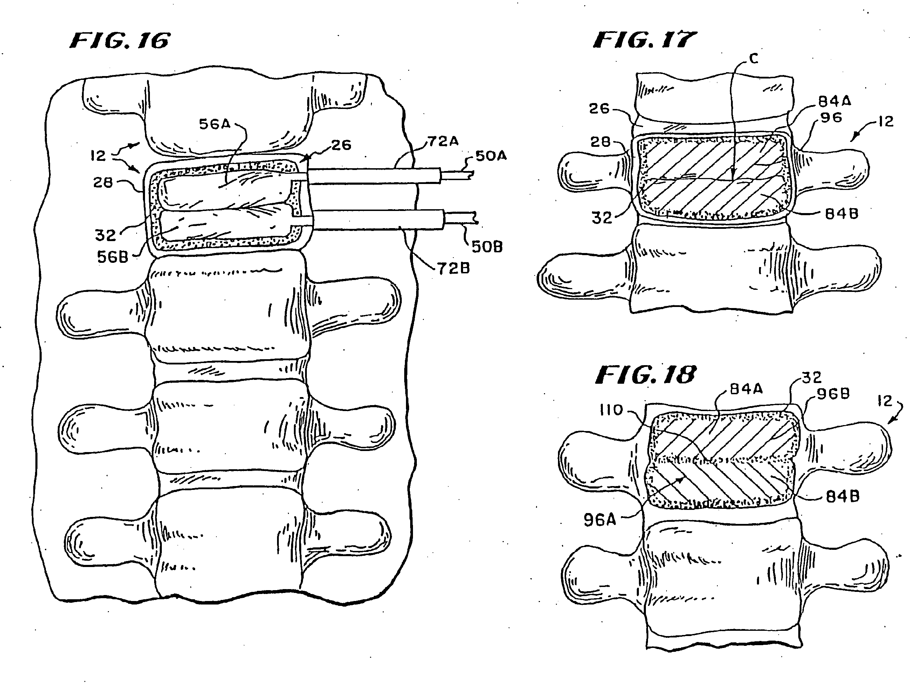

As another example, multiple expandable bodies 56A and 56B can access the vertebral body 26 from the same general region of the vertebra. Fig. 16 shows a representative dual ipsilateral posterolateral access, in which two outer guide sheaths 72A and 72B are used to provide separate access from the same lateral sides of the vertebral body 26.

Deployed from these access sites (see Fig. 17), the multiple expandable bodies 56A and 56B form vertically spaced, or stacked, cavities 84A and 84B. The vertically spaced cavities 84A and 84B may adjoin to form a single combined cavity (designated C in Fig. 17), into which the filling material 96 is injected. Alternatively (see Fig. 18), the vertically spaced cavities 84A and 84B may be separated by a region of cancellous bone (designated by numeral 110 in Fig. 18), forming multiple individual cavities 84A and 84B within the interior volume, each of which is individually filled with a filling material 96A and 96B.

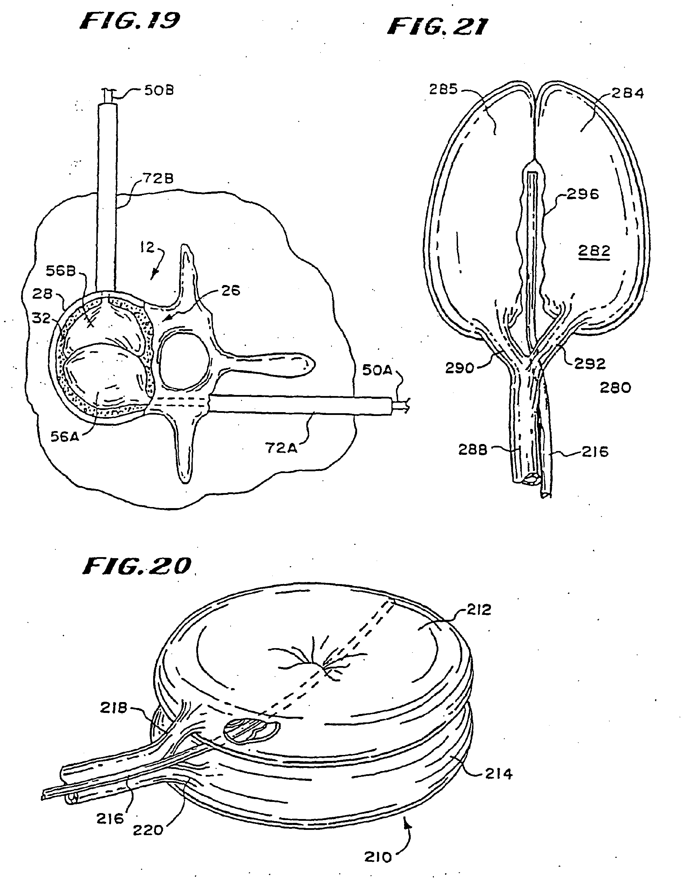

By way of another example, Fig. 19 shows a first outer guide sheath 72A arranged to provide a transpedicular access and a second outer guide sheath 72B to provide a posterolateral access.

Systems for treating bone using multiple expandable bodies can include directions 79 (see Fig. 12) for deploying the first and second expandable bodies. For example, the directions 79 can instruct the physician to insert a first expandable body into the interior volume through a first access path through cortical bone, while inserting a second expandable body into the interior volume through a second access path through cortical bone different than the first access path.

In any of the above-described examples, each guide sheath 72A or 72B can itself accommodate a single expandable body or multiple expandable bodies. The size and shape of the bodies may be the same, or they may vary, according to the desired objectives of the physician for the targeted vertebral body.

E. Representative Embodiments of Expandable Bodies to Treat Vertebrae

i. Constrained Donut-Shaped Geometries

Fig. 20 shows a representative embodiment of an expandable body, which is broadly denoted by the numeral 210. The body 210 comprises a pair of hollow, inflatable, non-expandable parts 212 and 214 of flexible material, such as PET or Kevlar. Parts 12 and 14 have a suction tube 216 therebetween for drawing fats and other debris by suction into tube 216 for transfer to a remote disposal location. The catheter tube 216 has one or more suction holes so that suction may be applied to the open end of tube 216 from a suction source (not shown).

The parts 212 and 214 are connected together by an adhesive which can be of any suitable type. Parts 212 and 214 are doughnut-shaped, as shown in Fig. 20 and have tubes 218 and 220 which communicate with and extend away from the parts 212 and 214, respectively, to a source of inflating liquid under pressure (not shown). The liquid expands the body 210 as already described.

Fig. 21 shows a modified doughnut shape body 280 of the type shown in Fig. 20, except the doughnut shapes of body 280 are not stitched onto one another. In Fig. 21, body 280 has a pear-shaped outer convex surface 282 which is made up of a first hollow part 284 and a second hollow part 285. A tube 288 is provided for directing liquid into the two parts along branches 290 and 292 to inflate the parts after the parts have been inserted into the interior volume of a bone. A catheter tube 216 may or may not be inserted into the space 296 between two parts of the balloon 280 to provide irrigation or suction. An adhesive bonds the two parts 284 and 285 together.

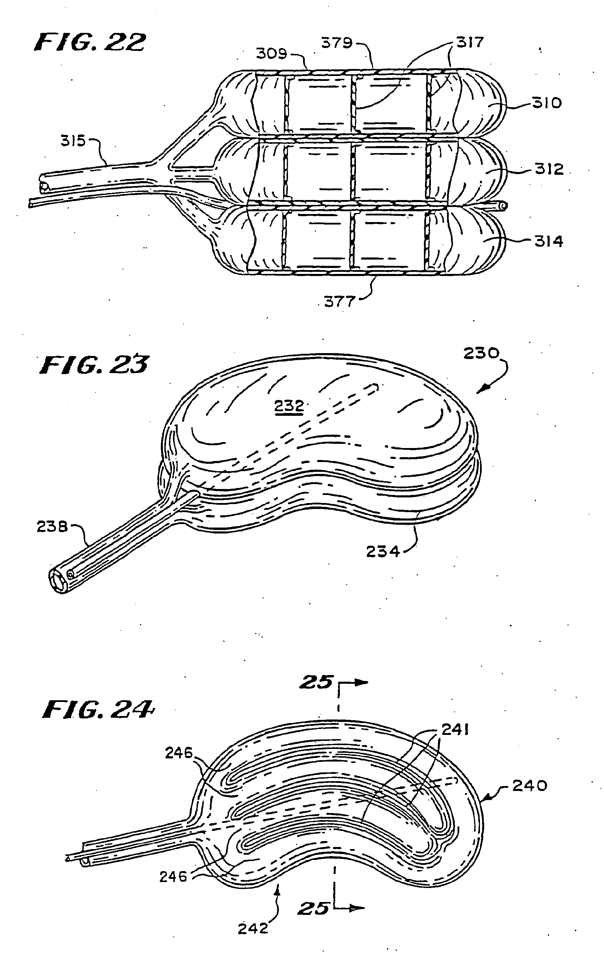

Fig. 22 shows another representative embodiment of an expandable body, designated 309. The body 309 has a generally round geometry and three expandable body units 310, 312 and 314. The body units 310, 312, and 314 include string-like external restraints 317, which limit the expansion of the body units 310, 312, and 314 in a direction transverse to the longitudinal axes of the body units 310, 312, and 314. The restraints 317 are made of the same or similar material as that of the body units 310, 312, and 314, so that they have some resilience but substantially no expansion capability.

A tubes 315 direct liquid under pressure into the body units 310, 312 and 314 to expand the units and cause compaction of cancellous bone. The restraints 317 limit expansion of the body units' prior to failure, keeping the opposed sides 377 and 379 substantially flat and parallel with each other.

**ii. constrained Kidney-Shaped Geometries**

Fig. 23 shows another representative embodiment of an expandable body 230, which has a kidney-shaped geometry. The body 230 has a pair of opposed kidney-shaped side walls 232 and a continuous end wall 234. A tube 238 directs liquid into the body to expand it within the vertebral body.

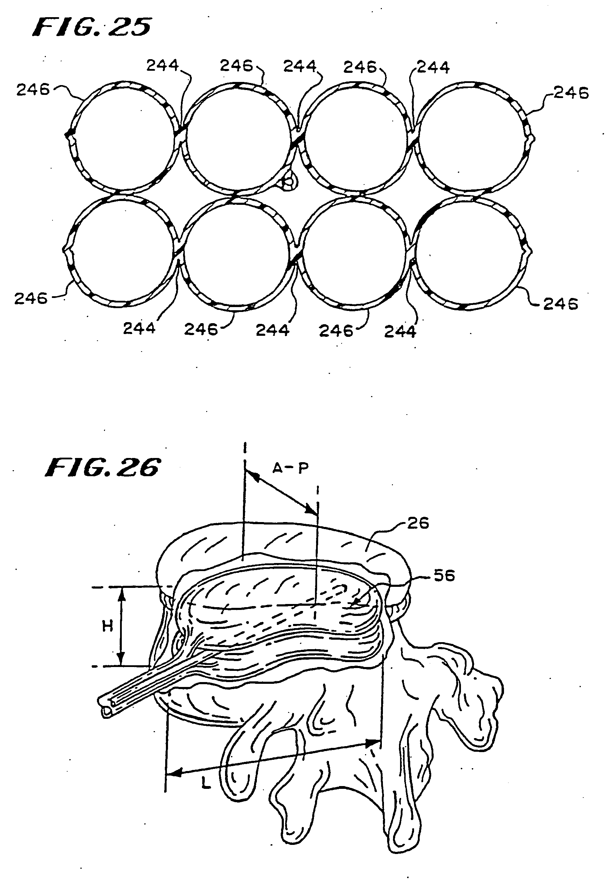

Fig. 24 shows another representative embodiment of an expandable body 242, which also has a kidney-shaped geometry. The body 242 is initially a single chamber bladder, but the bladder is branded along curved lines or strips 241 to form attachment lines 244 which take the shape of side-by-side compartments 246 which are kidney shaped as shown in Fig. 25. A similar pattern of strips as in 240 but in straight lines would be applied to a body that is square or rectangular. The branding causes a welding of the two sides of the bladder to occur.

The details of these and other expandable bodies usable to treat vertebral bodies are described in United States Patent Application, Serial No. 08/188,224, filed January 26, 1994.

**F. Selection of Desired Geometry**

The eventual selection of the size and shape of a particular expandable body or bodies to treat a targeted vertebral body 26 is based upon several factors. When multiple expandable bodies are used, the total combined dimensions of all expandable bodies deployed, when substantially expanded, should be taken into account.

The anterior-posterior (A-P) dimension (see Fig. 26) for the expandable body or bodies is selected from the CT scan or plain film or x-ray views of the targeted vertebral body 26. The A-P dimension is measured from the internal cortical wall of the anterior cortex to the internal cortical wall of the posterior cortex of the vertebral body. In general, the appropriate A-P dimension for the expandable body or bodies is less than this anatomic measurement.

The appropriate side to side dimension L (see Fig. 26) for an expandable body or bodies is also selected from the CT scan, or from a plain film or x-ray view of the targeted vertebral body. The side to side distance is measured between the internal cortical walls laterally across the targeted vertebral body. In general, the appropriate side to side dimension L for the expandable body is less than this anatomic measurement.

The lumbar vertebral body tends to be much wider in side to side dimension L then in A-P dimension. In thoracic vertebral bodies, the side to side dimension and the A-P dimensions are almost equal.

The height dimensions H of the expandable body or bodies (see Fig. 26) is chosen by the CT scan or x-ray views of the vertebral bodies above and below the vertebral body to be treated. The height of the vertebral bodies above and below the vertebral body to be treated are measured and averaged. This average is used to determine the appropriate height dimension of the chosen expandable body.

The dimensions of expandable body or bodies for use in vertebrae are patient specific and will vary across a broad range, as summarized in the following table:

| Vertebra Type | Height (H) Dimension of Typical Expandable Body or Bodies | Posterior (A-P) Dimension of Typical Expandable Body or Bodies | Side to Side Dimension (L) of Typical Expandable Body or Bodies |
|---|---|---|---|
| Lumbar | 0.5 cm to 4.0 cm | 0.5 cm to 4.0 cm | 0.5 cm to 5.0 cm |
| Thoracic | 0.5 cm to 3.5 cm | 0.5 cm to 3.5 cm | 0.5 cm to 4.0 cm |

A preferred expandable body 56 for use in a vertebral body is stacked with two or more expandable members of unequal height (see Fig. 26), where each member can be separately inflated through independent tube systems. The total height of the stack when fully inflated should be within the height ranges specified above. Such a design allows the fractured vertebral body to be returned to its original height in steps, which can be easier on the surrounding tissue, and it also allows the same balloon to be used in a wider range of vertebral body sizes.

## II. Treatment of Long Bones

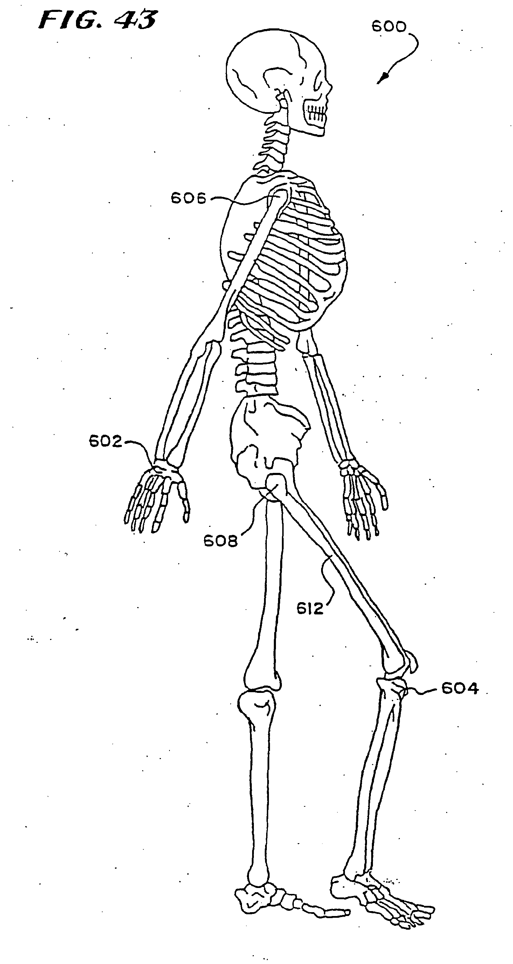

Like vertebrae, the interior regions of long bones substantially occupied by cancellous bone can be treated with the use of one or more expandable bodies. Fig. 43 shows representative regions of the human skeleton 600, where cancellous bone regions of long bones can be treated using expandable bodies. The regions include the distal radius (Region 602); the proximal tibial plateau (Region 604); the proximal humerus (Region 606); the proximal femoral head (Region 608); and the calcaneus (Region 610).

As for vertebral bodies, expandable bodies possess the important attribute of being able, in the course of forming cavities by compressing cancellous bone, to also elevate or push broken or compressed cortical bone back to or near its normal anatomic position. This is a particularly important attribute for the successful treatment of compression fractures or cancellous bone fractures in the appendicular skeleton, such as the distal radius, the proximal humerus, the tibial plateau, the femoral head, hip, and calcaneus.

Representative examples of expandable bodies for the treatment of cancellous bone regions of long bones will be next described.

## A. Expandable Body for the Distal Radius

The selection of an appropriate expandable to treat a fracture of the distal radius (Region 602 in Fig. 43) will depend on the radiological size of the distal radius and the location of the fracture.

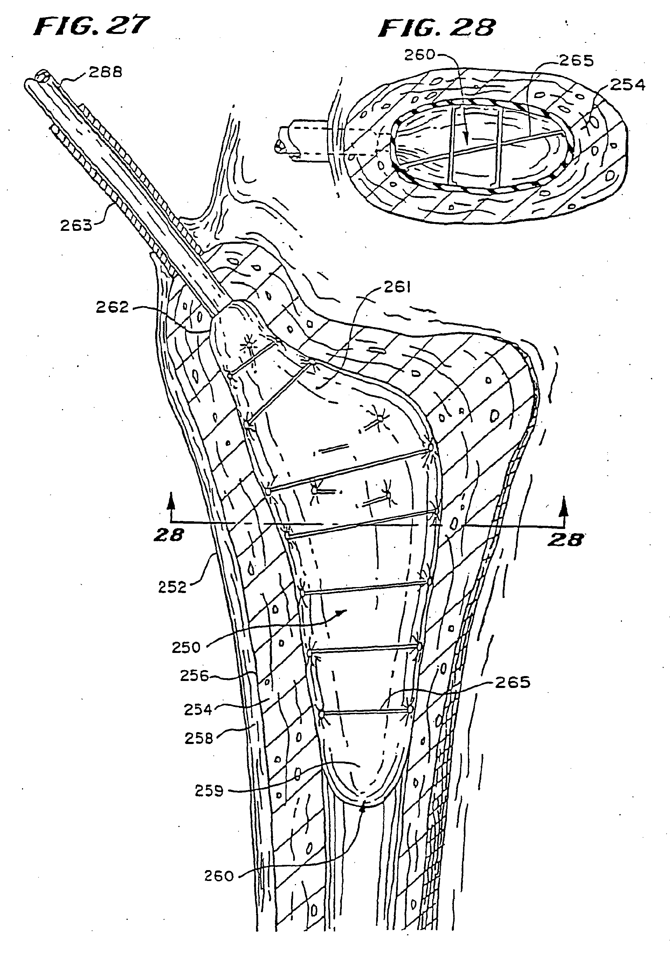

Figs. 27 and 28 show an expandable body 260 for use in the distal radius. The body 260, which is shown deployed in the distal radius 252 , has a shape which approximates a pyramid but more closely can be considered the shape of a humpbacked banana. The geometry of the body 260 substantially fills the interior of the space of the distal radius to compact cancellous bone 254 against the inner surface 256 of cortical bone 258.

The body 260 has a lower, conical portion 259 which extends downwardly into the hollow space of the distal radius 252. This conical portion 259 increases in cross section as a central distal portion 261 is approached. The cross section of the body 260 is shown at a central location (Fig. 27), which is near the widest location of the body 260. The upper end of the body 260, denoted by the numeral 262, converges to the catheter tube 288 for directing a liquid into the body 260 to expand it and force the cancellous bone against the inner surface of the cortical bone.

The shape of the body 260 is determined and restrained by tufts formed by string restraints 265. These restraints are optional and provide additional strength to the body 260, but are not required to achieve the desired configuration.

The body 260 is placed into and taken out of the distal radius in the same manner as that described above with respect to the vertebral bone.

Typical dimensions of the distal radius body vary as follows:

The proximal end of the body 260 (i.e. the part nearest the elbow) is cylindrical in shape and will vary from 0.4 x 0.4 cm to 1.8 x 1.8 cm.

The length of the distal radius body will vary from 1.0 cm to 12.0 cm.

The widest medial to lateral dimension of the distal radius body, which occurs at or near the distal radio-ulnar joint, will measure from 0.5 cm to 2.5 cm.

The distal anterior-posterior dimension of the distal radius body will vary from 0.4 to 3.0 cm.

## B. Expandable Body for Proximal Humerus Fracture

The selection of an appropriate expandable body 266 to treat a given proximal humeral fracture (Region 606 in Fig. 43) depends on the radiologic size of the proximal humerus and the location of the fracture.

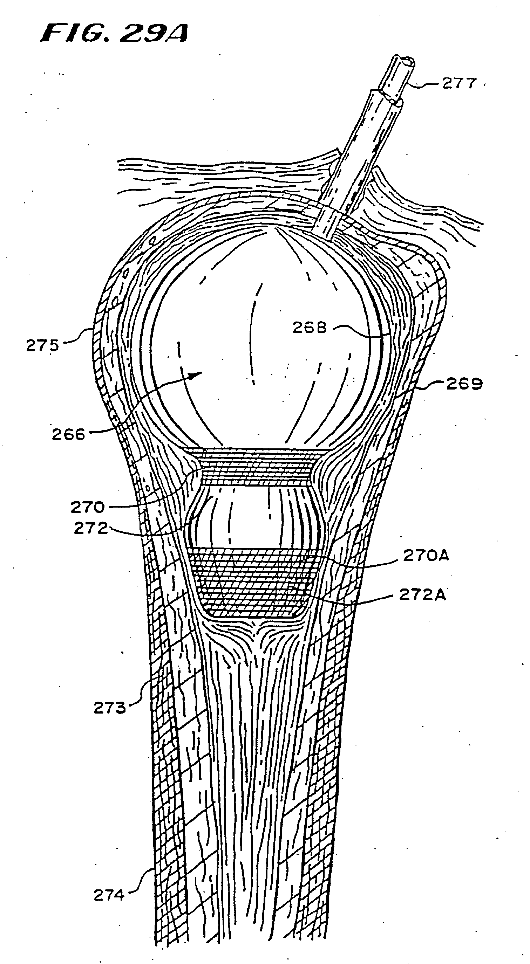

Fig. 29A shows an expandable body 266 for use in the proximal humerus 269. The body 266 is spherical for compacting the cancellous bone 268 in a proximal humerus 269. If surrounding cortical bone has experienced depression fracture, expansion of the body 266 also serves to elevate or move the fractured' cortical bone back to or near its anatomic position before fracture.

A mesh 270, embedded or laminated and/or winding, may be used to form a neck 272 on the body 266. A second mesh 270a may be used to conform the bottom of the base 272a to the shape of the inner cortical wall at the start of the shaft. These mesh restraints provide additional strength to the body 266, but the configuration can be achieved through molding of the body.

The body 266 has a catheter tube 277 into which liquid under pressure is forced into the body to expand it to compact the cancellous bone in the proximal humerus. The body 266 is inserted into and taken out of the proximal humerus in the same manner as that described above with respect to the vertebral bone.

Typical dimensions of the expandable body 266 shown in Fig. 29A for proximal humerus fracture vary as follows:

The spherical end of the body will vary from 0.6 x 0.6 cm to 3.0 x 3.0 cm.

The neck of the proximal humeral fracture body will vary from 0.5 x 0.5 cm to 3.0 x 3.0 cm.

The width of the base portion or distal portion of the proximal numeral fracture body will vary from 0.5 x 0.5 cm to 2.5 x 2.5 cm.

The length of the body will vary from 3.0 cm to 14.0 cm.

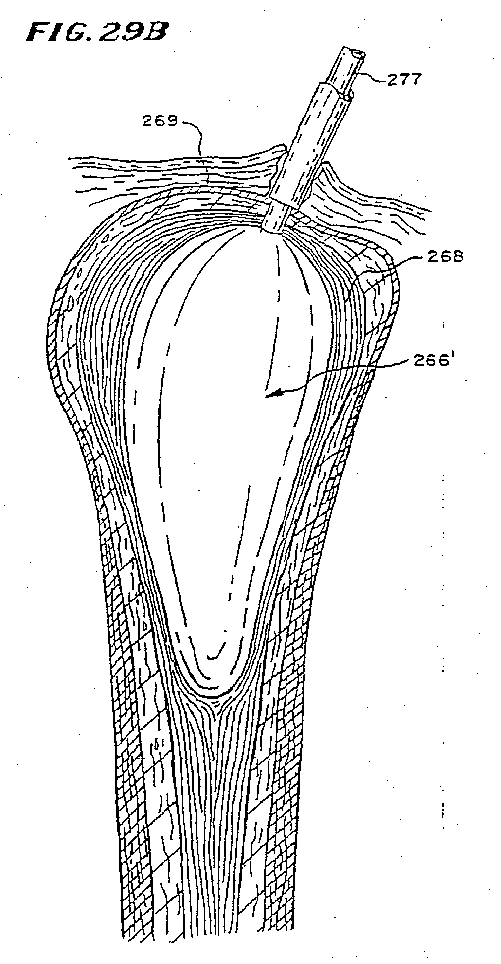

Fig. 29B shows another expandable body 266' for use in the proximal humerus 269. Instead of being spherical, the body 266' shown in Fig. 29B has a generally cylindrical geometry for compacting the cancellous bone 268 in a proximal humerus 269. Alternatively, the cylindrical body 266' can be elongated to form an elliptical or football-shaped geometry. Typical dimensions for a cylindrical or elliptical body vary from 0.6 cm to 3.0 cm in diameter to 3.0 cm to 14.0 cm in length.

## C. Expandable Body for Proximal Tibial Plateau Fracture

The selection of an expandable body to treat a given tibial plateau fracture (Region 604 in Fig. 43) will depend on the radiological size of the proximal tibial and the location of the fracture.

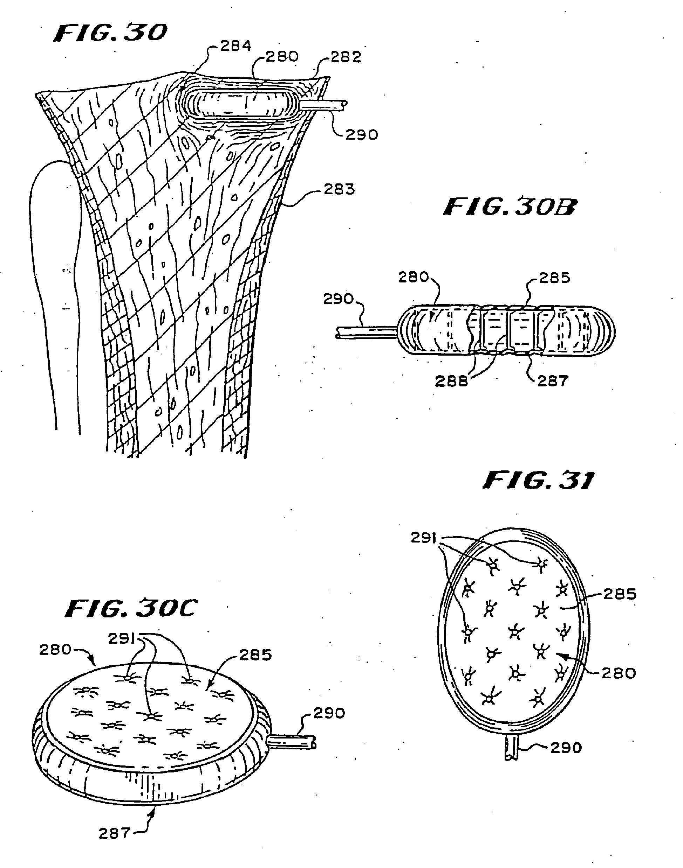

Fig. 30A shows an expandable body 280 for treating a tibial plateau fracture. The body 280 may be introduced into the tibia from any direction, as desired by the physician, for example, from the top, or medial, lateral, anterior, posterior, or oblique approach. In Fig. 30A, the body 280 has been introduced into cancellous bone 284 from the anterior side of the tibia 283 and is shown position in one side 282 of the tibia 283.

The body 280, when substantially inflated (as Fig. 30A shows), compacts the cancellous bone in the layer 284 surrounding the body 280. If the tibia plateau has experienced depression fracture, expansion of the body 280 also serves to move the tibia plateau back to or near its anatomic elevation before fracture, as Fig. 30A shows. Fractures on both the medial and lateral sides of the tibia can be treated in this manner.

As Fig. 30B shows, the body 280 has a pair of opposed sides 285 and 287. The sides 285 and 287 are interconnected by restraints 288, which pass through the body 280. Fig. 30C shows the tied-off ends 291 of the restraints 288.

The restraints 288 can be in the form of strings or flexible members of any suitable construction. The restraints 288 limit expansion of the body 280 prior to failure. The restraints 288 make the sides 285 and 287, when the body 280 is substantially expanded, substantially parallel with each other and, thereby, non-spherical.

A tube 290 is coupled to the body 280 to direct liquid into and out of the body to expand it. The body is inserted into and taken out of the tibia in the same manner as that described above with respect to the vertebral bone. Fig. 30C shows a substantially circular configuration for the body 280, although the body 280 can also be substantially elliptical, as Fig. 31 shows.

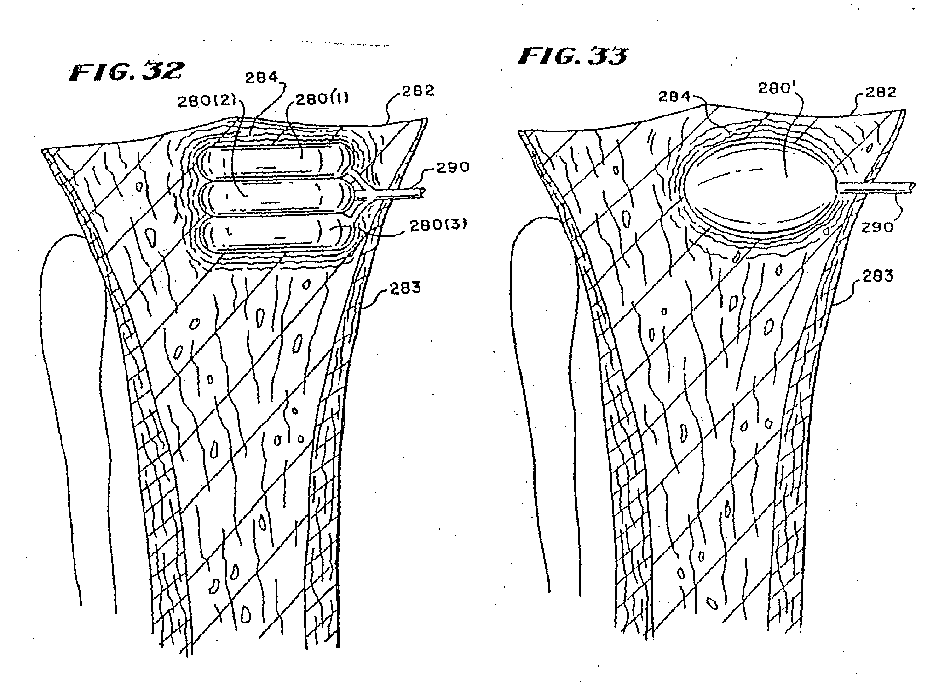

Other geometries and configurations can also be used. For example, as Fig. 32 shows, two or more expandable bodies 280(1), 280(2), and 280(3) can be stacked one atop another to produce a different cavity geometry and to enhance plateau fracture displacement. The multiple bodies 280(1), 280(2), and 280(3) can comprise separate units or be joined together for common deployment. When deployed as separate units, the bodies 280(1), 280(2), and 280(3) can enter through the same access point or from different access points.

As another example, as Fig. 33 shows, the body 280' can assume an egg shape when substantially inflated, to form a cavity and reshape broken bones. Other geometries, such as cylindrical or spherical, can also be used for the same purpose.

Typical dimensions of the body 280 for treating proximal tibial plateau fracture vary as follows:

The thickness or height of the body will vary from 0.3 cm to 5.0 cm.
The anterior-posterior (front to back) dimension will vary from 1.0 cm to 6.0 cm.
The medial to lateral (side-to-side) dimension will vary from 1.0 cm to 6.0 cm.

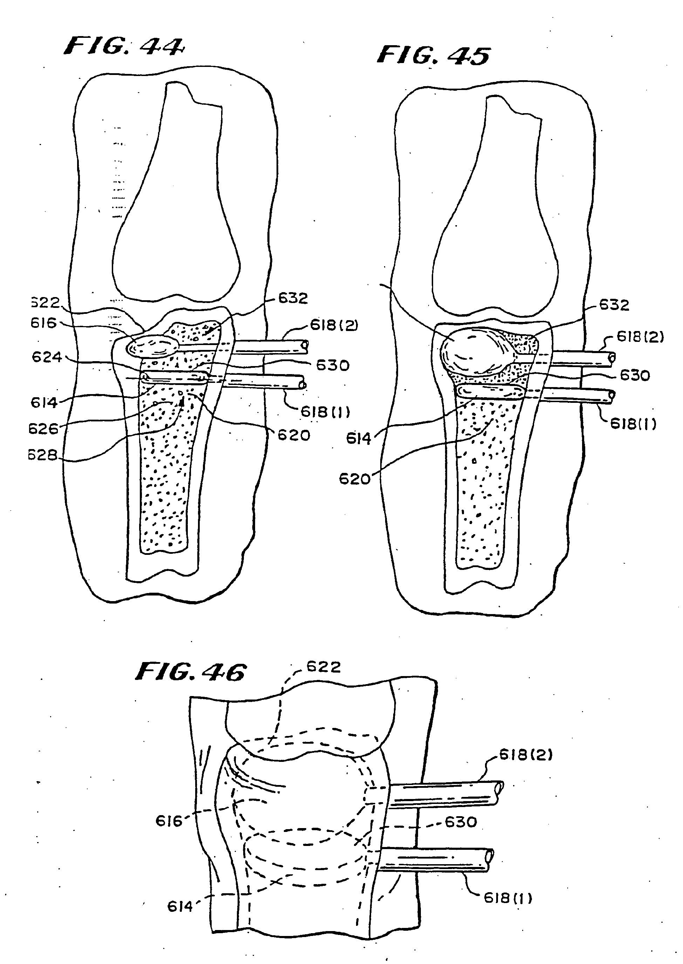

Figs. 44 and 45 show multiple expandable zones 614 and 616 deployed in cancellous bone 620. One zone 614 serves as a platform to confine and direct the expansion of the other zone 616. For the purpose of illustration, Figs. 44 and 45 show the multiple zones 614 and 616 used for this purpose to treat a tibial plateau fracture 622.

In the arrangement shown in Figs. 44 and 45, the zones 614 and 616 comprise separate expandable bodies. It should be appreciated, however, that the zone 614 and 616 can comprise parts of a single expandable body.

In the illustrated arrangement (as Fig. 44 shows), the first expandable body 614 is deployed through a first outer guide sheath 618(1) into cancellous bone 620 below the fracture 622. As Fig. 44 shows, when substantially expanded, the first body 614 expands more along its horizontal axis 624 (i.e., in a side-to-side direction) than along its vertical axis 626 (i.e., in an top-to-bottom direction). The greater expanded side-to-side geometry of the first body 614 compacts cancellous bone in a relatively thin region, which extends substantially across the interior volume 628 occupied by the first body 614. The geometric limits of the body 614 will typically fall just inside the inner cortical walls of the proximal tibia, or whatever bone in which the first body 614 is deployed.

The expanded first body 614 creates a barrier 630 within the interior region 628. Due to the less expanded top-to-bottom geometry of the first body 614, a substantially uncompacted region 632 of cancellous bone is left above the body 614, which extends from the formed barrier 630 upward to the fracture 622. In a representative deployment, the uncompacted region 632 extends about 2 cm below the tibial plateau fracture 622.

As Fig. 44 shows, a second expandable body 616 is deployed through a second outer guide sheath 618(2) into the uncompacted region 632 left between the first body 614, when substantially expanded, and the targeted tibial plateau fracture 622.

As Fig. 45 shows, the second expandable body 616 has a geometry, substantially like that shown in Figs. 30A to 30C. When substantially inflated, the second body 616 compacts a large percentage of the cancellous bone in the region 632 above the first expandable body 614. The presence of the barrier 630, which the expanded first body 614 creates (see Fig. 46 also), prevents expansion of the second body 616 in a direction away from the tibial platform fracture 622. Instead, the barrier 630 directs expansion of the second body 616 toward the fracture 622. Buttressed by the barrier 630, the expansion of the body 616 is directed against the fractured plateau 622, restoring it to its normal anatomic position, as Figs. 45 and 46 show.

It should be appreciated that one or more expandable bodies can be used as platforms or barriers to direct the expansion of one or more other expandable bodies in other localized interior bone regions. The barrier makes possible localized cavity formation in interior bone regions. Use of the barrier preserves healthy regions of cancellous bone, while directing the main compacting body toward localized fractures or localized regions of diseased cancellous bone.

D. Expandable Body for Femoral Head

The size of an expandable body for use in the femoral head (Region 608 in Fig. 43) is chosen based upon the radiological or CT scan size of the head of the femur and the location and size of the avascular necrotic bone.

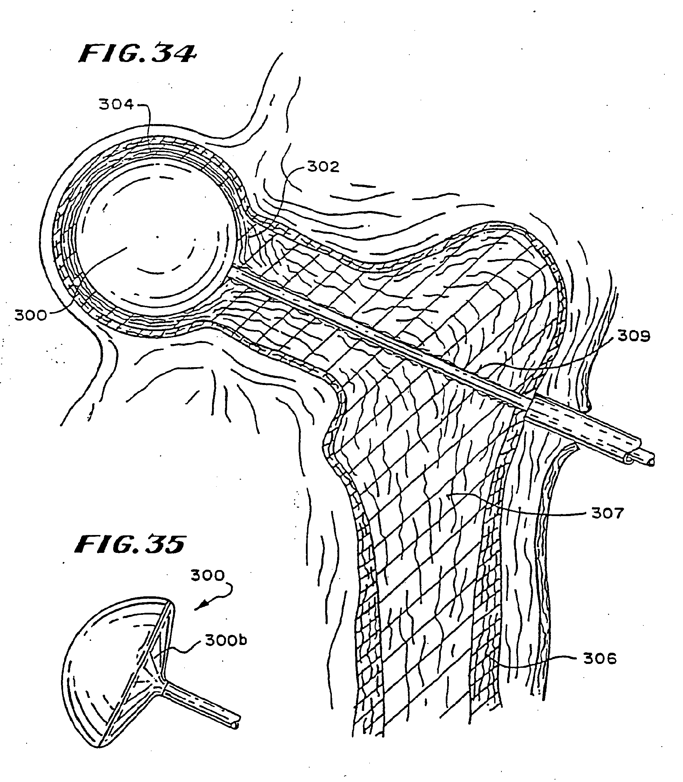

Fig. 34 shows an expandable body 300 introduced inside the cortical, bone 3.02 of the femoral head. As Fig.

34 shows, the femoral head is thin at the outer end 304 of the femur and increases in thickness at the lower end 306 of the femur. A tube 309 directs liquid to expand the body 300. The tube 309 extends along the femoral neck and into the femoral head. The expandable body 300 compacts the cancellous bone 307 in this bone region, while also moving fractured cortical bone back to or near its normal anatomic position.

The femoral head is generally spherical in configuration, and the body 300 can have either a hemispherical (see Fig. 35) as well as spherical geometry (as Fig. 34 shows) . The hemispherical shape is maintained in Fig. 34 by bonding overlapping portions of the body 300, creating pleats 300b.

The body 300 is inserted into and taken out of the femoral head in the same manner as that described with respect to the vertebral bone.

Typical dimensions of an expandable body for use in treating the femoral head vary as follows:

The diameter of the expandable body will vary from 0.5 cm to up to 4.5 cm. The dimensions of the hemispherical body (Fig. '35) are the same as the those of the spherical body (Fig. 34), except that approximately one half is provided.

**E. Expandable Body for Prevention of Hip Fracture**

Patients with bone density in the hip (Region 612 in Fig. 43) below a threshold value are at increased risk of hip fracture, and lower densities create greater risk. Patient selection is done through a bone density scan.

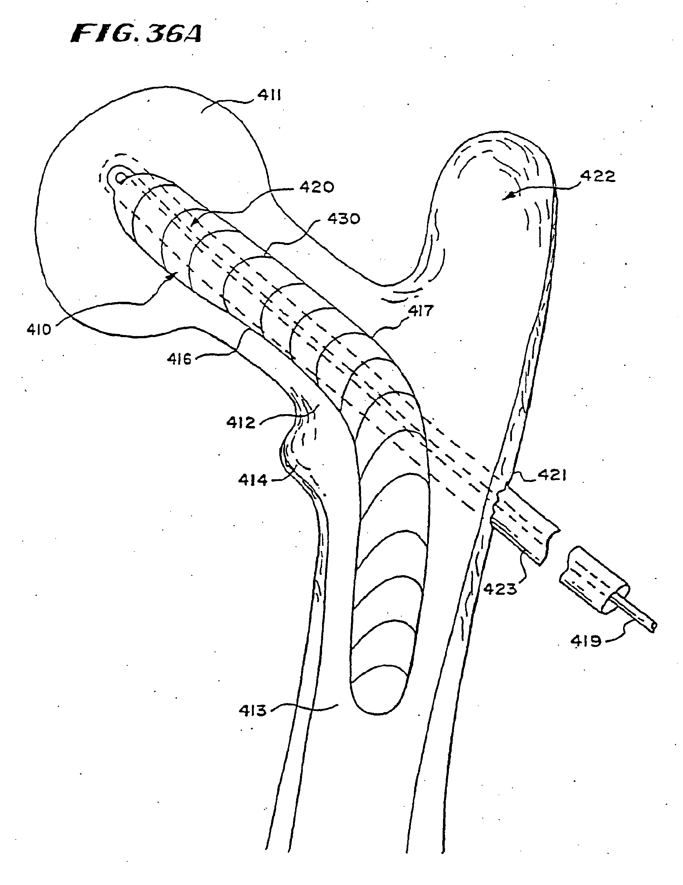

Fig. 36A shows an expandable body 410 having a "boomerang" geometry for use in preventing hip fracture. When substantially expanded (as Fig. 36A shows), the body 410 forms a cylinder, which gradually bends in the middle, like a boomerang, and extends from about 0.5 cm from the end of the femoral head 411 through the femoral neck 412 and down into the proximal femoral diaphysis 413 about 5 to 7 cm past the lesser trochanter 414.

Expansion of the body 410 is limited to achieve the described geometry by rings 430 of inelastic material. The rings 430 are held in a spaced apart relationship along one side of the body 410 by attachment to an inelastic band 416, which runs the length of that side of body 410. The rings 430 are held in a farther spaced apart relationship along the opposite side of the body 410 by attachment to another, longer inelastic band 417, which runs the length of the opposite side of the body 410. A tube 419 conveys liquid to inflate the body 410.

Prior to deployment within the body, the body 410 is collapsed and rolled up and held against the inflation tube 419 using, for example, with frangible connectors that will break as the body is subject to expansion. To deploy the body 410 into the hip, the surgeon uses a power drill under radiographic guidance to create a cavity 420, which is, for example, about 4 to 6 mm wide starting at the lateral femoral cortex 421 and proceeding into the femoral head 411. The body 410 is deployed through a guide sheath 423, following the cavity 420. The body 410 is deployed, prior to expansion, facing the lesser trochanter 414, so that expansion occurs toward the femoral diaphysis 413, and not toward the greater trochanteric region 422.

The expansion of the body 410 is guided by the rings 430 and bands 416 and 417, which cause bending of the body 410 downward into the lesser trochanter 414. Optionally, a second cavity can be drilled down into the diaphysis 413 , starting from the same entry point or from the other side.

The body length is chosen by the physician to extend about 0.5 cm from the end of the femoral head, through the femoral neck and into the proximal femoral diaphysis, usually about 4 to 8 cm below the lesser trochanter. The body diameter is chosen by measuring the inner cortical diameter of the femoral neck (the most narrow area) and subtracting 0.5 cm. The preferred dimensions of the body 410 are a total length of 10-20 cm and a diameter of about 1.0-2.5 cm.

Patients having the lowest bone densities in the femoral head may require greater compacting in the femoral head, which may, for example, be provided by using two bodies, one after the other: the bent body 410 followed by the femoral head body (inserted at the same point and expanded prior to inserting any supporting material). Alternatively, the bent body 410 may be adapted to have a distal portion that approximates the shape of the femoral head body.

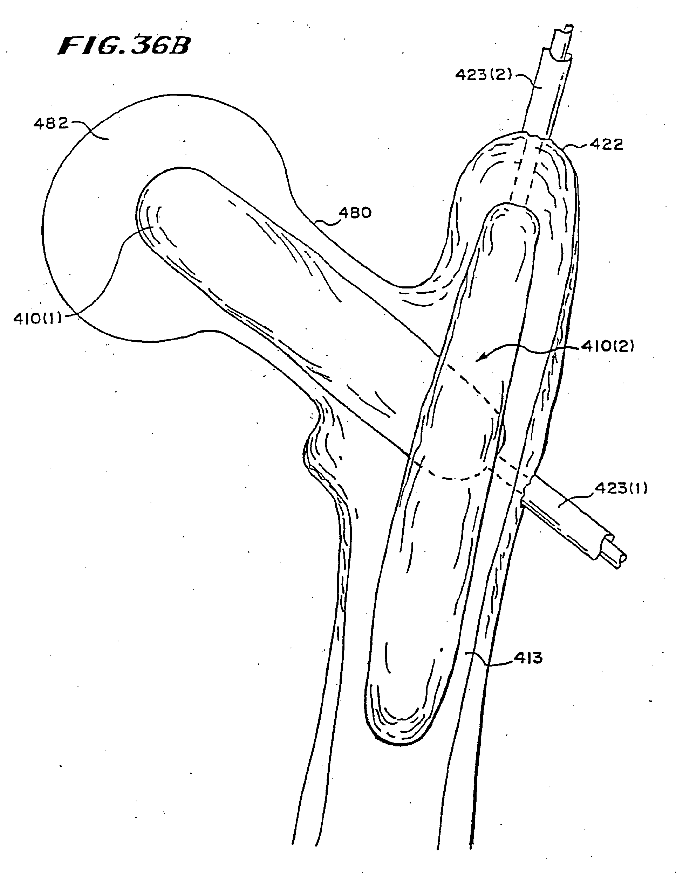

The geometry of the single, restrained body 410 can be approximated by multiple expandable bodies deployed separately, or coupled together, or stacked together. Fig. 36B shows the use of multiple expandable bodies in the hip region.

As Fig. 36B shows, a first expandable body 410(1) is introduced through a first outer guide sheath 423(1) in the proximal lateral cortex of the femoral shaft. The first body 419 (1) is deployed across the femoral neck 480 into the femoral head 482.

A second expandable body 410(2) is introduced through a second outer guide sheath 423 (2) in the greater trochanter 422 of the femur. The first body 419(1) is deployed in the direction of the femoral diaphysis 413.

Other approaches can be used. For example, one body can be introduced through the femoral neck 480, and the other body can be introduced along the shaft of the femur.

One or both of the bodies 410(1) and 410(2) can include external restraints to limit expansion, in the manner described with regard to the body 410. Expansion of the bodies 410(1) and 410(2) compacts cancellous bone to form a cavity having a geometry approximating that formed by the single body 410.

**F. Expandable Body for Calcaneus Fracture**

The size of an expandable body for use in treating fracture of the calcaneus (heel bone) (Region 610 in Fig. 43) is chosen based upon the radiological or CT scan size of the calcaneus and the location and size of the fracture.

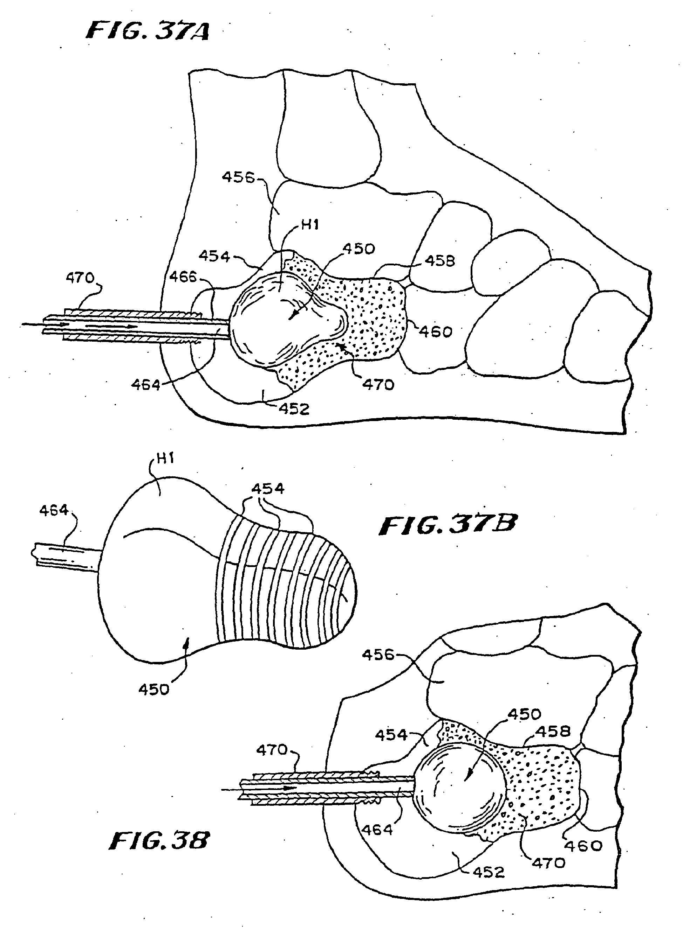

Figs. 37A and 37B show an expandable body 450 for treating fracture of the calcaneus 452. A tube 464 conveys liquid into the body 450 to expand it.

In Fig. 37A, the body 450 is deployed into the calcaneus 452 by a posterior approach, through the tuberosity. Other approaches can be used, as desired by the physician. A power drill opens a passage 466 through the tuberosity into the calcaneus. An outer guide sheath 470 is positioned within the passage 466, abutting the posterior of the calcaneus, in the manner previously described in obtaining access to a vertebral body. The body 450 is introduced through the guide sheath 470 and formed passage 466 into the calcaneus.

Expansion of the body 450 is limited within the confines of the calcaneus by inelastic peripheral bands 454 (see Fig. 37B). The bands 454 constrain expansion of the body 450 to an asymmetric, pear-shaped geometry, best shown in Fig. 37B. The pear-shaped geometry has a major dimension H1 occupying the region of the posterior facet 454. The major dimension H1 is located here, because the part of the calcaneus most likely to require elevation and realignment during expansion of the body 450 is the depressed part of the posterior facet 454 of the calcaneus, where the posterior facet 454 abuts the talus 456.

The pear-shaped geometry has a smaller, minor dimension occupying the region of the anterior facet 458 of the calcaneus, near the calcaneal-cuboid joint 460, between the calcaneus and cuboid bone 462.

Expansion of the body 410 compacts cancellous bone 470 within the calcaneus 452. The expansion also lifts a depression fracture of the posterior facet 454 back to or near its original anatomic elevation adjacent the talus 456. When collapsed and removed, the body 410 leaves a cavity in cancellous bone into which filling material can be introduced in the manner previously described.

Fig. 38 shows another expandable body 450' for use in treating fractures in the calcaneus. The body 450' in Fig. 38 has a more spherical or egg-shaped geometry than the pear-shaped body 450 shown in Fig. 37B. Like the pear-shaped body 450, the body 450', when expanded within the calcaneus, forms a cavity within cancellous bone and realigns fractured cortical bone at or near its normal anatomic position.

**III. Selection of Other Expandable Bodies (Further Overwiew)**

Different sizes and/or shapes of expandable bodies may be used at sites not specified above, such as the jaw bones, the midshaft of the arm and leg bones, the cervical vertebral bodies, the foot and ankle bones, the pelvis, the ribs, and the like.

The choice of the shape and size of a expandable body takes into account the morphology and geometry of the site to be treated. As before stated, the shape of the cancellous bone to be compressed, and the local structures that could be harmed if bone were moved inappropriately, are generally understood by medical professionals using textbooks of human skeletal anatomy along with their knowledge of the site and its disease or injury. Precise dimensions for a given patient can be further determined by X-ray of the site to be treated.

As one general guideline, the selection of the geometry of the expandable body should take into account that at least 40% of the cancellous bone volume needs to be compacted in cases where the bone disease causing fracture (or the risk of fracture) is the loss of cancellous bone mass (as in osteoporosis) . The preferred range is about 30% to 90% of the cancellous bone volume. Compacting less of the cancellous bone volume can leave too much of the diseased cancellous bone at the treated site. The diseased cancellous bone remains weak and can later collapse, causing fracture, despite treatment.

Another general guideline for the selection of the geometry of the expandable body is the amount that the targeted fractured bone region has been displaced or depressed. The expansion of the body within the cancellous bone region inside a bone can elevate or push the fractured cortical wall back to or near its anatomic position occupied before fracture occurred.

However, there are times when a lesser amount of cancellous bone compaction is indicated. For example, when the bone disease being treated is localized, such as in avascular necrosis, or where local loss of blood supply is killing bone in a limited area, the expandable body can compact a smaller volume. This is because the diseased area requiring treatment is smaller.

Another exception lies in the use of an expandable body to improve insertion of solid materials in defined shapes, like hydroxyapatite and components in total joint replacement. In these cases, the body shape and size is defined by the shape and size of the material being inserted.

Yet another exception is the delivery of therapeutic substances, which will be described in greater detail later. In this case, the cancellous bone may or may not be diseased or adversely affected. Healthy cancellous bone can be sacrificed by significant compaction to improve the delivery of a drug or growth factor which has an important therapeutic purpose. In this application, the size of the expandable body is chosen by the desired amount of therapeutic substance sought to be delivered. In this case, the bone with the drug inside is supported while the drug works, and the bone heals through exterior casting or current interior or exterior fixation devices.

Generally speaking, providing relatively inelastic properties for the expandable body, while not always required, is nevertheless preferred when maintaining a desired shape and size within the bone is important, for example, in bone graft placement or in a vertebral body,' where the spinal cord is nearby. Using relatively inelastic bodies, the shape and size can be better predefined; taking into account the normal dimensions of the outside edge of the cancellous bone. Use of relatively inelastic materials also more readily permits the application of pressures equally in all directions to compress cancellous bone. Still, substantially equivalent results can usually be achieved by the use of multiple expandable bodies having highly elastic properties, if expansion is controlled by either internal or external restraints, as previously disclosed.

### IV. optimal Orientation with Asymmetric structures

### A. Conventional Symmetric structures

Fig. 50 shows a tool 848, which includes a catheter tube 850 having a proximal and a distal end, respectively 52 and 54. The catheter tube 850 includes a handle 851 to facilitate gripping and maneuvering the tube 850. The handle 851 is preferably made of a foam material secured about the catheter tube 850.

The distal end 854 carries an expandable structure 856, which Fig. 51 shows to be of conventional construction used, e.g., in the deployment in veins and arteries, e.g., in angioplasty applications. The structure 856 is shown in Fig. 50 in a substantially collapsed geometry. The structure 856 conventionally comprises an elongated tube, formed, for example, by standard polymer extrusion and molding processes. The tubular structure 856 is bonded at its opposite ends 858 to the catheter tube 850, using, for example, an adhesive. When substantially collapsed, the structure 856 can be inserted into an interior body region.

Fig. 51 shows an enlarged view of the structure 856 when in a substantially expanded geometry. As Fig. 51 shows, the middle region 864 of the tubular structure 856, when substantially expanded, assumes a generally cylindrical shape, which is symmetric about the main axis 860 of the catheter tube 850. Expansion stretches the polymer material of the structure 856 near its bonded ends 858 to form generally conical end portions 862.

When the structure 856 is inserted into bone in accordance with the teachings of the above described U.S. Patents 4,969,888 and 5,108,404, the access portal 843 (see Figs. 52 and 53) extends at an angle downward toward the bottom of the vertebral body 826 to enter the interior volume 830. As Figs. 52 and 53 show, the access portal 843 aligns the catheter tube axis 860 obliquely with respect to all natural axes 866, 867, or 869 of the vertebral body 826.

As the conventional structure 856 expands within the interior volume 830 (as Figs. 54 and 55 show, respectively, in lateral and coronal views), the structure 856 symmetrically expands about the catheter tube axis 860, compressing cancellous bone 832 to form a cavity 868. However, since the catheter tube axis 860 is oriented obliquely relative to all natural axes 866, 867, or 869, the formed cavity is not centered with respect to the middle region MR. Instead, the cavity 868 is offset on one lateral side of the middle region MR (as Fig. 55 shows) and also extends from top to bottom at oblique angle through the middle region MR (as Fig. 54 shows).

Due to these asymmetries, the cavity 868 will not provide optimal support to the middle region MR when filled with bone cement. Since the bone cement volume is not centered about the middle region MR, the capability of the vertebral body 826 to withstand loads is diminished. The asymmetric compaction of cancellous bone 832 in the interior volume 830 may also exert unequal or nonuniform interior forces upon cortical bone 832, making it difficult to elevate or push broken and compressed bone.

### B. Optimal Orientation for Cancellous Bone Compaction

Fig. 56A shows an improved bone treating tool 814, which includes a catheter tube 816 carrying at its distal end 818 an expandable structure 820. The catheter tube 816 can, at its proximal end, be configured like the tube 850 shown in Fig. 50, with a handle 851 made of, e.g., a foam material.

Fig. 56A shows the structure 20 in a substantially expanded condition, in which the structure comprises a cylinder 821 with generally conical portions 834, each having a top 825 and a base 827. The tops 825 of conical portions 834 are secured about the catheter tube 816 and, in this respect, are generally aligned with the catheter tube axis 824. However, unlike the expandable structure 856 shown in Fig. 51, the main axis 822 of the cylinder 821 and the axis 824 of the catheter tube 816 are not aligned. Instead, the cylinder axis 822 is offset at an angle A from the catheter tube axis 824. As a result, the structure 820, when substantially expanded (as Fig. 56A shows), is not symmetric with respect to the catheter tube axis 824.

In Fig. 56A, the bases 827 of the conical portions 834 extend generally perpendicularly to the cylinder axis 822.

In this orientation, the tops 825 and the bases 827 are not parallel to each other. Other orientations are possible. For example, in Fig. 56B, the bases 827 of the conical portions 834 extend generally perpendicularly to the catheter tube axis 824. In this orientation, the tops 825 and the bases 827 are generally parallel to each other.

Fig. 57 shows in lateral view, the offset structure 820 shown in Fig. 56A deployed in the interior volume 830 of a vertebral body 826. As before shown in Figs. 54 and 55, the approach in Fig. 57 does not align the catheter tube axis 824 with any of the natural axes 866, 867, and 869 of the body 826. However, as Fig. 57 shows, the expansion of the offset cylinder 821 of the structure 820 about its axis 822 is not symmetric with respect to the catheter tube axis 824. Instead, expansion of the offset structure 820 is generally aligned with the natural axes 866 and 869 of the vertebral body 826. As Fig. 57 shows, the single offset structure 820 can form a cavity 838 that, while still laterally offset to one side of the middle region MR (as shown in Fig. 55), is nevertheless symmetric in a top-to-bottom respect with the middle region MR. A matching, adjacent cavity can be formed by deployment of a second offset structure 820 on the opposite lateral side of the vertebral body 826. The composite cavity, formed by the two offset bodies 820, introduced simultaneously or in succession by dual access, is substantially centered in all respects about the middle region MR.

A cavity centered with respect to the middle region MR provides support uniformly across the middle region MR when filled with bone cement. The capability of the vertebral body 826 to withstand loads is thereby enhanced. The symmetric compaction of cancellous bone 832 in the interior volume 830 that a centered cavity provides also exerts more equal and uniform interior forces upon cortical bone 832, to elevate or push broken and compressed bone.

Figs. 58A and 58B show an expandable structure 1200 having an offset, asymmetric geometry different than the geometry of the offset expandable structure 820 shown in Figs. 56A and 56B. In Figs. 58A and 58B, the offset angle A between the cylinder axis 822 and the catheter tube axis 824 is an acute angle. As a result, the axis 822 of the structure 820 is offset in a nonparallel dimension or plane relative to the catheter tube axis 824. In Figs. 58A and 58B, the offset angle A between the cylinder axis 1220 and the catheter tube axis 1240 is zero, as the axis 1220 of the cylinder 1210 is offset at a distance from and in a generally parallel dimension or plane relative to the catheter tube axis 1240. The catheter tube 160 can, at its proximal end, be configured like the tube 50 shown in Fig. 50, with a handle 51 made of, e.g., a foam material.

As in Figs. 56A and 56B, the tops 1250 of conical portions 1040 are secured about the catheter tube 160 and, in this respect, are generally aligned with the catheter tube axis 1240. In Figs. 58A and 58B, the orientation of the bases 1270 of the conical portions 1040 differ. In Fig. 58A, the bases 1270 of the conical portions 1040 extend generally perpendicularly to the catheter tube axis 1240, and are therefore generally parallel to the tops 1250 (comparable to the orientation shown in Fig. 56B) . In Fig. 58B, the bases 1270 of the conical portions 1040 extend at an angle B to the catheter tube axis 1240. In this orientation, the tops 1250 and the bases 1270 are not parallel to each other.

Figs. 56A and 56B and 58A and 58B show that it is possible, by adjustment of the offset angle A, as well as adjustment of the orientation of the conical end bases, to achieve virtually any desired offset geometry, and thereby tailor the orientation of the expandable structure to the particular geometry of the point of use.

C. Maximizing Cancellous Bone Compaction

Referring back to Fig. 51, when the conventional tubular structure 856 shown in Fig. 51 is substantially expanded, material of the structure is stretched into conical sections 862 near the ends 858, which are bonded to the catheter tube 850. Fig. 59 shows the geometry of expanded tubular structure 856 in greater detail. The conical portions 862 extend at a cone angle $\alpha$ from the bonded ends 858. The expanded structure 856 therefore presents the generally cylindrical middle region 864, where the maximum diameter of the structure 856 ($BODY_{DIA}$) exists, and the conical portions 862, which comprise regions of diameter that decreases with distance from the middle region 864 until reaching the diameter of the catheter tube ($TUBE_{DIA}$) .

Due to the geometry shown in Fig. 59, maximum cancellous bone compaction does not occur along the entire' length (L2) of the conventional structure 856, as measured between the bonded ends 858. Instead, maximum cancellous bone compaction occurs only along the effective length (L1) of the cylindrical middle region 864 of the structure 856, where the structure 856 presents its maximum diameter $BODY_{DIA}$. Cancellous bone compaction diminishes along the length of the conical portions 862, where the structure's diameter progressively diminishes. At the bonded ends 858, and portions of the catheter tube 850 extending beyond the bonded ends 858, no bone compaction occurs. The catheter tube 850 can, at its proximal end, be configured like the tube 850 shown in Fig. 50, with a handle 851 made of, e.g., a foam material.

The lengths (Lc) of the conical regions 862 and bonded ends 858 relative to the entire length of the structure 856 (L2) are important indications of the overall effectiveness of the structure 856 for compacting cancellous bone. The effective bone compaction length (L1) of any expandable structure having conical end regions, such as structure 856 shown in Fig. 59, can be expressed as follows:

$$L1 = L2 - 2(Lc)$$

where the length of a given conical region (Lc) can be expressed as follows:

$$Lc = \frac{h}{\tan\frac{\alpha}{2}}$$

where:

$$h = \frac{BODY_{DIA} - TUBE_{DIA}}{2}$$

where (see Fig. 59):

$BODY_{DIA}$ is the maximum diameter of the middle region 864, when substantially expanded,
$TUBE_{DIA}$ is the diameter of the catheter tube 850, and
$\alpha$ is the angle of the conical portion.

As the foregoing expressions demonstrate, for a given conical angle $\alpha$, the length Lc of the conical portions 862 will increase with increasing maximum diameter $BODY_{DIA}$ of the middle region 864. Thus, as $BODY_{DIA}$ is increased, to maximize the diameter of the formed cavity, the lengths Lc of the conical portions 862 also increase, thereby reducing the effective length L1 of maximum cancellous bone compaction.

The bone compaction effectiveness of an expandable structure of a given maximum diameter increases as L1 and L2 become more equal. The geometry of a conventional tubular structure 856 shown in Fig. 59 poses a tradeoff between maximum compaction diameter and effective compaction length. This inherent tradeoff makes optimization of the structure 856 for bone compaction application difficult.

Fig. 60 shows an improved structure 870 having a geometry, when substantially expanded, which mitigates the tradeoff between maximum compaction diameter and effective compaction length. The structure 870 includes a middle region 872, where $BODY_{DIA}$ occurs. The structure 870 also includes end regions 874, which extend from the middle region 872 to the regions 876, where the material of the structure is bonded to the catheter tube 878, at $TUBE_{DIA}$. The catheter tube 878 can, at its proximal end, be configured like the tube 850 shown in Fig. 50, with a handle 851 made of, e.g., a foam material.

In the arrangement shown in Fig. 60, the end regions 874 are molded or stressed to provide a non-conical diameter transformation between $BODY_{DIA}$ and $TUBE_{DIA*}$ The diameter changes over two predefined radial sections $r_1$ and $r_2$, forming a compound curve in the end regions 874, instead of a cone. The non-conical diameter transformation of radial sections $r_1$ and $r_2$ between $BODY_{DIA}$ and $TUBE_{DIA}$ reduces the differential between the effective bone compaction length L1 of the structure 870 and the overall length L2 of the structure 870, measured between the bond regions 876.

Fig. 61 shows another improved expandable structure 880 having a geometry mitigating the tradeoff between maximum compaction diameter and effective compaction length. Like the structure 870 shown in Fig. 60, the structure 880 in Fig. 61 includes a middle region 882 of $BODY_{DIA}$ and end regions 884 extending from the middle region to the bonded regions 886, at $TUBE_{DIA}$. As the structure 870 in Fig. 60, the end regions 884 of the structure 880 make a non-conical diameter transformation between $BODY_{DIA}$ and $TUBE_{DIA}$. In Fig. 61, the predefined radial sections $r_1$ and $r_2$ are each reduced, compared to the radial section $r_1$ and $r_2$ in Fig. 60. As a result, the end regions 884 take on an inverted profile. As a result, the entire length L2 between the bonded regions 886 becomes actually less than the effective length L1 of maximum diameter $BODY_{DIA}$. The catheter tube can, at its proximal end, be configured like the tube 850 shown in Fig. 50, with a handle 851 made of, e.g., a foam material.

The structures 870 and 880, shown in Figs. 60 and 61, when substantially inflated, present, for a given overall length L2, regions of increasingly greater proportional length L1, where maximum cancellous bone compaction occurs.

Furthermore, as in Fig. 61, the end regions 884 are inverted about the bonded regions 886. Due to this inversion, bone compaction occurs in cancellous bone surrounding the bonded regions 886. Inversion of the end regions 884 about the bonded regions 886 therefore makes it possible to compact cancellous bone along the entire length of the expandable structure 880.

Fig. 62 shows another improved expandable structure 890. Like the structure 880 shown in Fig. 61, the structure 890 includes a middle region 892 and fully inverted end regions 894 overlying the bond regions 896. The structure 880 comprises, when substantially collapsed, a simple tube. At least the distal end of the tubular structure 880 is mechanically tucked or folded inward and placed into contact with the catheter tube 898. As shown in Fig. 62, both proximal and distal ends of the tubular structure are folded over and placed into contact with the catheter tube 898. The catheter tube 898 can, at its proximal end, be configured like the tube 850 shown in Fig. 50, with a handle 851 made of, e.g., a foam material.

The catheter tube 898 is dipped or sprayed beforehand with a material 1102 that absorbs the selected welding energy, for example, laser energy. The folded-over ends 894 are brought into abutment against the material 1102. The welding energy transmitted from an external source through the middle region 992 is absorbed by the material 1102. A weld forms, joining the material 1102 , the folded-over ends 894, and the catheter tube 850. The weld constitutes the bond regions 896.

The inverted end regions 894 of the structure 890 achieve an abrupt termination of the structure 890 adjacent the distal end 1104 of the catheter tube 898, such that the end regions 894 and the distal catheter tube end 1104 are coterminous. The structure 890 possesses a region of maximum structure diameter, for maximum cancellous bone compaction, essentially along its entire length. The structure 890 presents no portion along its length where bone compaction is substantially lessened or no cancellous bone compaction occurs.

Figs. 63 and 64 show another arrangement of an expandable structure 1110. As Fig. 64 shows, the structure 1110 includes a middle region 1112 of maximum diameter $BODY_{DIA}$ and inverted end regions 1114, which overlie the bonded regions 1116.

Fig. 63 shows the structure 1110 before the end regions 1114 have been inverted in the manufacturing process. As Fig. 63 shows, the structure 1110 comprises, when substantially collapsed, a simple tube. To facilitate formation of the inverted end regions 1114 and bonded regions 1116, a two-piece catheter tube is provided, comprising an outer catheter tube' 1118 and an inner catheter tube 1120. The inner catheter tube 1120 slides within the outer catheter tube 1118. The catheter tube 1118 can, at its proximal end, be configured like the tube 50 shown in Fig. 50, with a handle 851 made of, e.g., a foam material.

As Fig. 63 shows, during the manufacturing process, the inner catheter tube 1120 is moved a first distance d1 beyond the outer catheter tube 1118. In this condition, the proximal and distal ends 1122 and 1124 of the tubular structure 1110 are bonded, without folding over or tucking in, about the inner catheter tube 1118 and the outer catheter tube 1120, respectively. The unfolded ends 1122 and 1124 of the tubular structure 1110 can then be directly exposed to conventional adhesive or melt bonding processes, to form the bonded regions 1116.

Once the bonded regions 1116 are formed, the inner catheter tube 1120 is moved (see arrow 1130 in Fig. 64) to a distance d2 (shorter than d1) from the end of the outer catheter tube 1118. The shortening of the inner tube 1120 relative to the outer tube 1120 inverts the ends 1122 and 1124. The inversion creates double jointed end regions 1116 shown in Fig. 64, which overlie the bonded regions 1116. The relative position of the outer and inner catheter tubes 1118 and 1120 shown in Fig. 64 is secured against further movement, e.g., by adhesive, completing the assemblage of the structure 1110.

The double jointed inverted ends 1114 of the structure 1110 in Fig. 64, like single jointed inverted ends 894 of the structure 890 in Fig. 62, assure that no portion of the catheter tube protrudes beyond the expandable structure. Thus, there is no region along either structure 894 or 1114 where cancellous bone compaction does not occur. Like the structure 890 shown in Fig. 62, the structure 1110 in Fig. 20 presents a maximum diameter for maximum cancellous bone compaction essentially along its entire length.

Fig. 65 shows another arrangement of an improved expandable structure 1300 well suited for deployment in an interior body region. Like the structure 1110 shown in Figs. 63 and 64, the structure 1300 in Fig. 65 includes an inner catheter tube 1304 secured within an outer catheter tube 1302. Like the structure 1110 shown in Figs. 63 and 64, the distal end 1310 of the inner catheter tube 1304 in Fig. 65 extends beyond the distal end 1308 of the outer catheter tube 1302.

The outer diameter of the inner catheter tube 1304 is likewise smaller than the inner diameter of the outer catheter tube 1302. A flow passage 1312 is defined by the space between the two' catheter tubes 1302 and 1304.

The proximal end 1314 of an expandable body 1306 is bonded to the distal end 1308 of the outer catheter tube 1302. The distal end 1316 of the expandable body 1306 is bonded to the distal end 1310 of the inner catheter tube 1304. An inflation medium 1318 is conveyed into the body 1306 through the flow passage 1312, causing expansion of the body 1306.

In Fig. 65, the physical properties of the structure 1300 at the proximal body end 1314 differ from the physical properties of the structure 1300 at the distal body end 1316. The different physical properties are created by material selection. More particularly, materials selected for the inner catheter tube 1304 and the expandable body 1306 are more compliant (i.e., more elastic) than the materials selected for the outer catheter tube 1302. In a preferred arrangement, materials selected for the expandable body 1306 and the inner catheter tube 1304 possess hardness properties of less than about 90 Shore A and ultimate elongation of greater than about 450%, e.g., more compliant polyurethanes. In a preferred arrangement, materials selected for the outer catheter tube 1302 possess hardness properties of greater than about 45 Shore D and ultimate elongation of less than about 450%, e.g., less compliant polyurethanes or polyethylenes.

Due to the differential selection of materials, the lack of compliance of the outer catheter tube 1302 at the proximal body end 1314 is counterpoised during expansion of the body 1306 against the compliance of the inner catheter tube 1304 at the distal body end 1316. The different compliance characteristics causes the body 1306, during expansion, to increase in length in proportion to its increase in diameter during expansion. By virtue of the more compliant body 1306 and inner catheter tube 1304, the structure 1300 shown in Fig. 65 is elastic enough to conform to an interior body region, like inside a bone. Nevertheless, the structure 1300 is constrained from overexpansion by attachment of the proximal end 1314 of the body 1306 to the less elastic outer catheter tube 1302.

The bond between a given expandable structure and its associated catheter tube can be strengthened by using a $CO_2$ or NdYAG laser to weld the structure and tube materials together. Factors influencing joint strength include energy wave length, energy pulse width, pulse period, head voltage, spot size, rate of rotation, working distance, angle of attack, and material selection.

The catheter tube 1302 can, at its proximal end, be configured like the tube 850 shown in Fig. 50, with a handle 851 made of, e.g., a foam material.

## D. Deployment in the Vasculature

Fig. 66 shows a blood vasculature region 1400. The region 1400 includes a first blood vessel 1402, which extends along a first axis 1404. The region 1400 also includes a second blood vessel 1406, which branches from the first blood vessel 1402 along a second axis 1408 offset from the first axis 1404.

Fig. 66 also shows the presence of an occlusion 1410 adjacent the second blood vessel 1406. The occlusion 1410 can comprise, e.g., plaque buildup along the interior wall of the second blood vessel 1406.

Fig. 67 shows the distal end of a tool 1412, which has been introduced into the vascular region 1400 for the purpose of opening the occlusion 1410. The tool 1412 comprises a catheter tube 1416, which carries at its distal end an expandable structure 1420 of the type shown in Fig. 56A. The catheter tube 1416 can, at its proximal end, be configured like the tube 850 shown in Fig. 50, with a handle 851 made of, e.g., a foam material.

The catheter tube 1416 is introduced by conventional vascular introducer and, with fluoroscopic monitoring, steered to the targeted region 1400 along a guidewire 1430 deployed within the first and second vessels 1402 and 1406. The structure 1420 is expanded using a sterile fluid, like saline or a radio-contrast medium. Fig. 67 shows the structure 1420 in a substantially expanded condition.

Like the expandable structure 820 shown in Fig. 56A, the main axis 1422 of the structure 1420 shown in Fig. 67 and the axis 1424 of the catheter tube 1416 are not aligned. Instead, the structure axis 1422 is offset at a selected acute angle A from the catheter tube axis 1424. Due to the offset angle A, the structure 1420, when substantially expanded (as Fig. 67 shows), is not symmetric with respect to the catheter tube axis 1424.

As Fig. 67 shows, the asymmetric expansion of the structure 1420 allows the physician to maintain the catheter tube 1416 in axial alignment with the first blood vessel 1402, while maintaining the expandable structure 1420 in axial alignment with the second blood vessel 1406. In this orientation, expansion of the structure 1420 within the second blood vessel 1406 opens the occlusion 1410. The asymmetry of the structure 1420 relative to the catheter tube 1416 thereby permits access to branched blood vessels without complex manipulation and steering.

## E. Deflection of the structure

In all of the foregoing embodiments, a length of the associated catheter tube extends within the expandable structure. In the embodiments shown in Figs. 51, 56A/B, 58A/B, and 59 to 62, the enclosed catheter tube comprises an extension of the main catheter tube. In the embodiments shown in Figs. 63 to 65, the enclosed catheter tube comprises a separate catheter tube carried by the main catheter tube.

Regardless of the particular construction (see Fig. 68), the enclosed length of catheter tube 1600 provides an interior lumen 1602 passing within the expandable structure 1604. The lumen 1602 accommodates the passage of a stiffening member or stylet 1606 made, e.g., from stainless steel or molded plastic material.

The presence of the stylet 1606 serves to keep the structure 1604 in the desired distally straightened condition during passage through an associated guide sheath 1608 toward the targeted body region 1610, as Fig. 68 shows. Access to the target body region 1610 through the guide sheath 1608 can be accomplished using a closed, minimally invasive procedure or with an open procedure.

As shown in Fig. 69, the stylet 1606 can have a preformed memory, to normally bend the distal region 1612 of the stylet 1606. The memory is overcome to straighten the stylet 1606 when confined within the guide sheath 1608, as Fig. 68 shows. However, as the structure 1604 and stylet 1606 advance free of the guide sheath 1608 and pass into the targeted region 1610, the preformed memory bends the distal stylet region 1612. The bend of the distal stylet region 1612 bends the tube 1600 and thereby shifts the axis 1614 of the attached expandable structure 1604 relative to the axis 1616 of the access path (i.e., the guide sheath 1608). The prebent stylet 1606, positioned within the interior of the structure 1604, further aids in altering the geometry of the structure 1604 in accordance with the orientation desired when the structure 1604 is deployed for use in the targeted region 1610.

## V. SINGLE USE

Expansion of any one of the expandable structures described herein during first use in a targeted body region generates stress on the material or materials which make up the structure The material stress created by operational loads during first use in a targeted body region can significantly alter the molded morphology of the structure, making future performance of the structure unpredictable.

For example, expansion within bone during a single use creates contact with surrounding cortical and cancellous bone. This contact can damage the structure, creating localized regions of weakness, which may escape detection. The existence of localized regions of weakness can unpredictably cause overall structural failure during a subsequent use.

In addition, exposure to blood and tissue during a single use can entrap biological components on or within the structure or the associated catheter tube. Despite cleaning and subsequent sterilization, the presence of entrapped biological components can lead to unacceptable pyrogenic reactions.

As a result, following first use, the structure can not be relied upon to reach its desired configuration during subsequent use and may not otherwise meet established performance and sterilization specifications. The effects of material stress and damage caused during a single use, coupled with the possibility of pyrogen reactions even after resterilization, reasonably justify imposing a single use restriction upon devices which carry these expandable structures for deployment in bone.

To protect patients from the potential adverse consequences occasioned by multiple use, which include disease transmission, or material stress and instability, or decreased or unpredictable performance, the invention also provides a kit 1500 (see Figs. 70 and 71) for storing a single use probe 1502, which carries an expandable structure 1504 described herein prior to deployment in bone.

In the illustrated embodiment (see Figs. 70 and 71), the kit 1500 includes an interior tray 1508. The tray 1508 holds the probe 1502 in a lay-flat, straightened condition during sterilization and storage prior to its first use. The tray 1508 can be formed from die cut cardboard or thermoformed plastic material. The tray 1508 includes one or more spaced apart tabs 1510, which hold the catheter tube 1503 and expandable structure 1504 in the desired lay-flat, straightened condition. As-shown, the facing ends of the tabs 1510 present a nesting, serpentine geometry, which engages the catheter tube 1503 essentially across its entire width, to securely retain the catheter tube 1503 on the tray 1508.

The kit 1500 includes an inner wrap 1512, which is peripherally sealed by heat or the like, to enclose the tray 1508 from contact with the outside environment. One end of the inner wrap 1512 includes a conventional peal-away seal 1514 (see Fig. 71), to provide quick access to the tray 1508 upon instance of use, which preferably occurs in a sterile environment, such as within an operating room.

The kit 1500 also includes an outer wrap 1516, which is also peripherally sealed by heat or the like, to enclosed the inner wrap 1512. One end of the outer wrap 1516 includes a conventional peal-away seal 1518 (see Fig. 71), to provide access to the inner wrap 1512, which can be removed from the outer wrap 1516 in anticipation of imminent use of the probe 1502, without' compromising sterility of the probe 1502 itself.

Both inner and outer wraps 1512 and 1516 (see Fig. 71) each includes a peripherally sealed top sheet 1520 and bottom sheet 1522. In the illustrated embodiment, the top sheet 1520 is made of transparent plastic film, like polyethylene or MYLAR™ material, to allow visual identification of the contents of the kit 1500. The bottom sheet 1522 is made from a material that is permeable to EtO sterilization gas, e,g., TYVEC™ plastic material (available from DuPont).

The sterile kit 1500 also carries a label or insert 1506, which includes the - statement "For Single Patient Use Only" (or comparable language) to affirmatively caution against reuse of the contents of the kit 1500. The label 1506 also preferably affirmatively instructs against resterilization of the probe 1502. The label 1506 also preferably instructs the physician or user to dispose of the probe 1502 and the entire contents of the kit 1500 upon use in accordance with applicable biological waste procedures. The presence of the probe 1502 packaged in the kit 1500 verifies to the physician or user that probe 1502 is sterile and has not be subjected to prior use. The physician or user is thereby assured that the expandable structure 1504 meets established performance and sterility specifications, and will have the desired configuration when expanded for use.

**VI. Confinement of Filling Material**

**A. Dual Stage Filling**

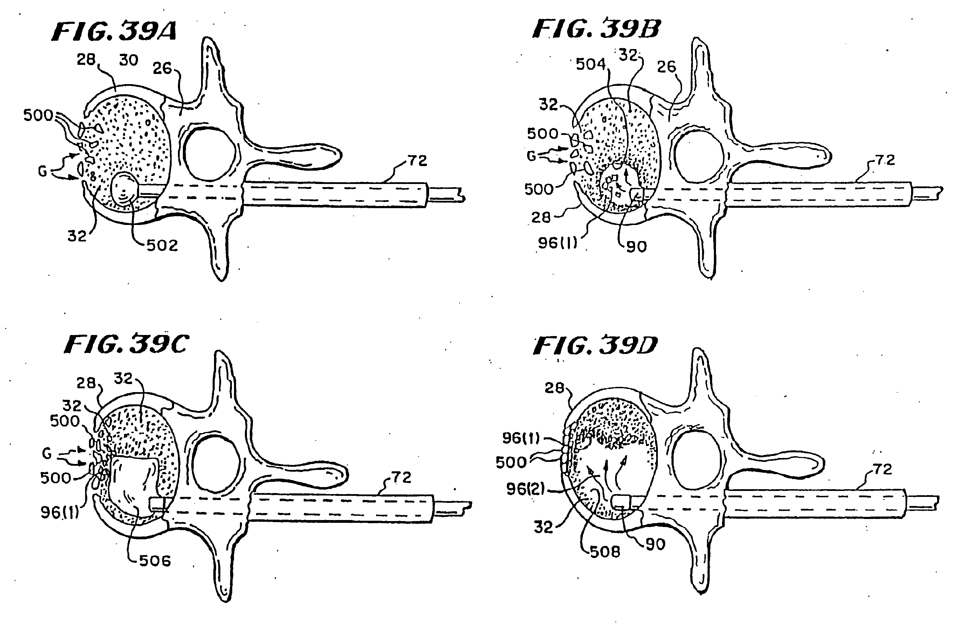

Figs. 39A to 39D show a multiple stage process for introducing filling material into a cavity formed by an expandable body in cancellous bone. The process is shown in association with treating a vertebral body. This is for the purpose of illustration. It should be appreciated that the process can be used in the treatment of all bone types.

Use of the multi-stage process is indicated when pre-examination of the targeted bone reveals that a portion of the cortical wall 28 has fractured or failed (as Fig. 39A shows at the anterior region of the vertebral body 26). The failed cortical wall 28 creates gaps and cracks (designated G in Fig. 39A). Typically, remnant chips 500 of the failed cortical bone 28 may lay in the cancellous bone 32 in the region where cortical wall failure has occurred. Filling material can flow or seep through these gaps or cracks C outside of the interior volume of the bone.

The process begins at the point where the outer guide sheath 72 has been positioned and the guide pin removed in the manner previously described. The physician introduces a first expandable body 502 into the cancellous bone 32 near the failed cortical bone region, as Fig. 39A shows. The first expandable' body 502 is sized, when substantially expanded, to occupy a relatively small volume (i.e., less than about 20%) of the volume of cancellous bone 32 in interior volume 30.

The physician expands the first expandable body 502, compacting a relatively small region of cancellous bone. Upon collapse and removal of the first body 502, a small cavity 504, caused by the compaction, remains (as Fig. 39B shows).

The physician introduces the injector tip 90 and injects an aliquot of filling material 96(1) (for example, about 1 cc to about 9 cc) into the formed small cavity 504 (as Fig. 39B shows).

In a short time interval (before the filling material 96 (1) is allowed to substantially set and harden), the physician withdraws the injector tip 90 and introduces a second expandable body 506 into the cancellous bone 32 (see Fig. 39C). The second expandable body 506 is larger than the first body 502. The second body 506 is sized to create the desired geometry for the therapeutic main cavity 508 in cancellous bone 32.

As Fig. 39C shows, expansion of the second body 506 displaces the earlier injected aliquot of filling material 96(1) in the cavity 504 toward the failed cortical wall region. The aliquot of filling material 96(1) will envelop remnant chips' 500 of cortical bone lying in its path. The material 96(1) and enveloped chips 500 are pressed against the failed cortical bone region as expansion of the second body 506 progresses. The first aliquot of filling material 96(1) will begin to set and harden as the main therapeutic cavity 508 is being formed by the expansion of the second body 506. The second body 506 is collapsed and removed, leaving the main cavity 508.

As Fig. 39D shows, the first aliquot of filling material 96(1) provides a viscous or (in time) hardened boarder region along the anterior edge of the cavity 508. As subsequent injection of additional filling material 96(2) into the main cavity 508 proceeds, as Fig. 39D shows, the viscous or hardened boarder region 96(1) impedes passage of the additional filling material 96(2) as it fills the main cavity 508. The viscous or hardened boarder region 96(1) serves as a dam, keeping the additional filling material 96(2) entering the main cavity 508 from seeping from the vertebral body 26.

B. Interior Mesh

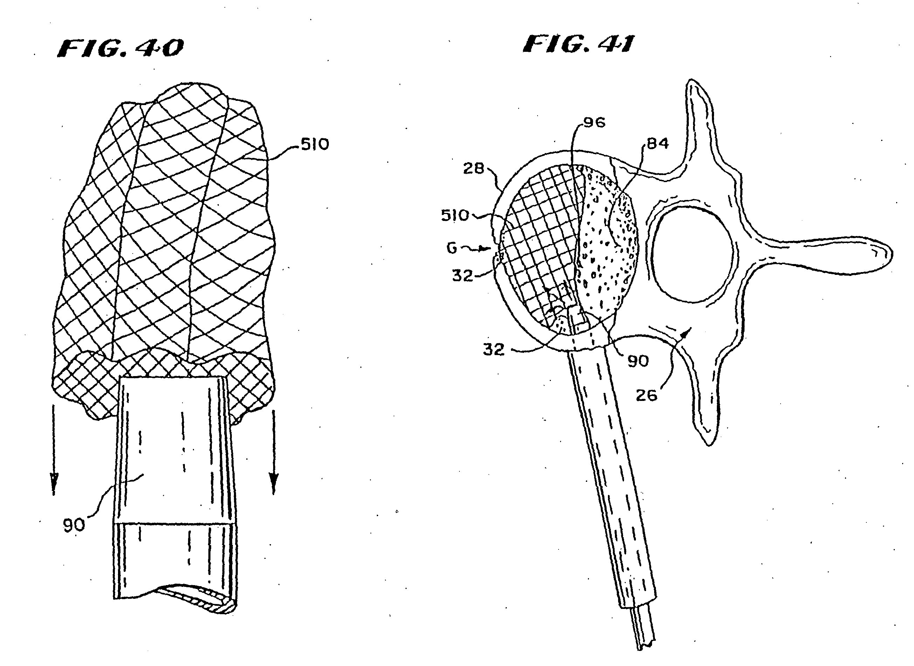

Figs. 40 and 41 show the use of an interior mesh 510 in association with the introduction of filling material into a cavity formed by an expandable body in cancellous bone. The mesh 510 is shown in association with treating a vertebral body, but it should be appreciated that the process can be used in the treatment of all bone types.

Use of the mesh 510 is indicated when pre-examination of the targeted bone reveals a failed cortical bone region (as Fig. 41 shows at the anterior region of the vertebral body 26), coupled with the lack of enough bone matter, due to advanced disease or a complex fracture, to adequately fill the failed cortical bone region by compacting using an expandable body. Flowable cement material can flow or seep through the unfilled gaps or cracks (designated G in Fig. 41) present in the failed cortical bone region.

The mesh 510 comprises a woven structure made from biocompatible material like Goretex™ material, Nitinol™ material, or Dacron™ material. The mesh presents a surface area, which is about 1/3rd to 1/2 of the interior area of the main therapeutic cavity 84 formed by the selected expandable body.

Before deploying the injector tip 90 into the' formed cavity 84 (which is deployed in Fig. 41 by posterolateral access), the physician drapes the mesh 510 over the tip 90, as Fig. 40 shows. As Fig. 41 shows, the viscous flow of filling material 96 injected from the tip 90 carries the mesh 510 into the cavity 84 in advance of the filling material 96. The mesh 510 is urged by the filling material 96 into contact with the anterior region of the bone, including the failed cortical bone region. The mesh 510, permeated with viscous material 96 and resting over the failed cortical bone region, impedes passage of filling material, until hardening occurs.

VII. Delivery of Therapeutic Materials

A cavity created in cancellous bone by any of the expandable bodies described above can be filled with a medically-appropriate formulation of a drug or a growth factor.

An expandable body can compact infected cancellous bone to create a space which can be filled with the antibiotic gel in an open or minimally invasive procedure. The cavity places and holds the required amount of drug right at the site needing treatment, and protects the drug from being washed away by blood or other fluids.

Not only can the dose be optimized; but additional doses can be applied at later times without open surgery, enhancing the therapeutic outcome. If the required cavity for the optimal drug dose weakens the bone, the bone can be protected from future fracture with a cast or with current internal or external metal or plastic fixation devices.

The therapeutic substance put into bone may act outside the bone as well. A formulation containing chemotherapeutic agent could be used to treat local solid tumors, localized multiple myeloma or even a nearby osteosarcoma or other tumor near that bone.

The cavity formed by an expandable body can be filled with an appropriate supporting material, like acrylic bone cement or biocompatible bone substitute, which carries a therapeutic substance. Alternatively, the therapeutic substance can be separately delivered before injection of the filling material. Thus, using an expandable body, the physician is able to treat a fracture while also delivering a desired therapeutic substance (like an antibiotic, bone growth facer or osteoporosis drug) to the site.

As an alternative, to deliver therapeutic substances, bodies can be dipped in a medical formulation (often a dry powder, liquid or gel) containing a medically-effective amount of any desired antibiotic, bone growth factor or other therapeutic agent to coat the body with the above-mentioned substance before it is inserted into a bone being treated. Optionally, the body can be wholly or partially expanded before the coating is performed. Optionally, the coated body can be dried with air or by other means when the applied formulation is wet, such as a liquid or a gel. The body is refolded as required and either used immediately or stored, if appropriate and desired. Coated on the body, therapeutic substances can be delivered while cancellous bone is being compressed, or with an additional body once the cavity is made.

The methods described above can also be used to coat Gelfoam or other agents onto the body before use. Inflating the Gelfoam-coated body inside bone will further fill any cracks in fractured bone not already filled by the compressed cancellous bone.

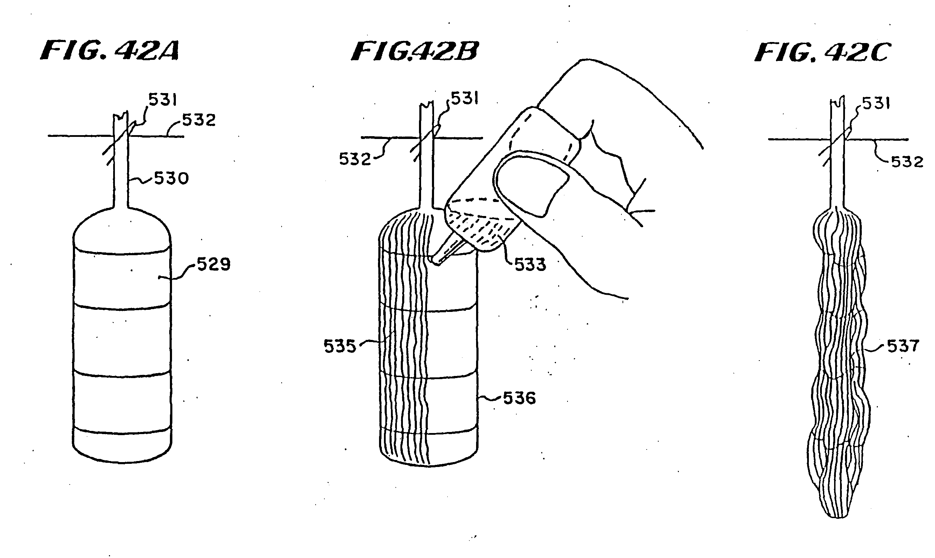

Figs. 42A to 42C schematically illustrate one system and method for delivering a therapeutic substance to the bone using an expandable body 529. The body 529 is carried at the end of the catheter tube 530, which conveys liquid to expand the body 529, as previously described.

As shown in Fig. 42A, the expandable body 529, in a substantially expanded condition, is stabilized with a clip 531 that couples the catheter tube 530 to a wire 532. As shown in Fig. 42B, a measured amount of gel formulation containing the desired amount of substance 533 is uniformly dispensed from a container 534, preferably in thin lines 535, onto the outer surface of the body 536. The coating substance can be the desired compound alone in its natural state (solid, liquid or gas) or in an appropriate formulation, including a dry powder, an aerosol or a solution. As shown in Fig. 42C, the coated body 537 is collapsed and allowed to dry until the gel sets. Alternatively, the body 536 can also be coated without prior expansion. The optional drying time will, of course, depend on the nature of the compound and its formulation. The coated body 237 is suitable for packaging for use by a surgeon.

Delivering a therapeutic substance on the outside of expandable body used to compact the bone, or with an expandable body introduced after the bone is compacted, is qualitatively different than putting formulated drug into the cavity. When delivered while the bone is compressed, the therapeutic substance becomes incorporated into the compacted bone. This can serve as a way to instantly formulate a slow release version of the substance.

The cavity formed by the expandable body can be filled with an appropriate supporting material, like acrylic bone cement or biocompatible bone substitute, as before described.

Medically-effective amounts of therapeutic substances are defined by their manufacturers or sponsors and are generally in the range of 10 nanograms to 50 milligrams per site, although more or less may be required in a specific case.

For example, the cavity can accommodate a typical dose of the antibiotic, gentamicin, to treat a local osteomyelitis (bone infection). A typical dose is about 1 gram, although the therapeutic range for gentamicin is far greater, from 1 nanogram to 100 grams, depending on the condition being treated and the size of the area to be covered. A medically-suitable gel formulated with appropriate gel materials, such as Polyethylene glycol, can contain 1 gram of gentamicin in a set volume of gel, such as 10 cc.

Other antibiotics that can be used to treat bone infection include, for example, ancef, nafcillin, erythromycin, tobramycin, and gentamicin. Typical bone growth factors are members of the Bone Morphogenetic Factor, Osteogenic Protein, Fibroblast Growth Factor, Insulin-Like Growth Factor and Transforming Growth Factor alpha and beta families. Chemotherapeutic and related agents include compounds such as cisolatin, doxcrubicin, daunorubicin, methotrexate, taxol and tamoxifen. Osteoporosis drugs include estrogen, calcitonin, diphosphonates, and parathyroid hormone antagonists.

VIII. Delivery of Biomaterials

A cavity created in cancellous bone by any of the expandable bodies described above can also be filled with biomaterials.

Biomaterials which do not flow into the formed cavity, like hydroxyapatite granules or bone mineral matrix, can be pushed down a tube with a long pin whose diameter is slightly more narrow than the inner-diameter of the outer guide sheath, using the minimally-invasive procedure. During open surgery, the physician can approach the bone in the same way.

If the biomaterial to be inserted does not flow and should not be pushed into the cavity through the guide sheath (as in the case of the hydroxyapatite block, because that can cause damage), the physician can form the cavity using a minimally invasive approach, then punch a hole using standard tools (such as a punch, gouge or rasp) into one side of the cortical bone to allow insertion of the block.

IX. Bone Marrow Harvesting

Any of the expandable bodies described above can also be used in the harvesting of bone marrow for diagnostic or therapeutic purposes, for example, in the diagnosis of multiple myeloma or in the treatment of advanced cancers with bone marrow transplants.

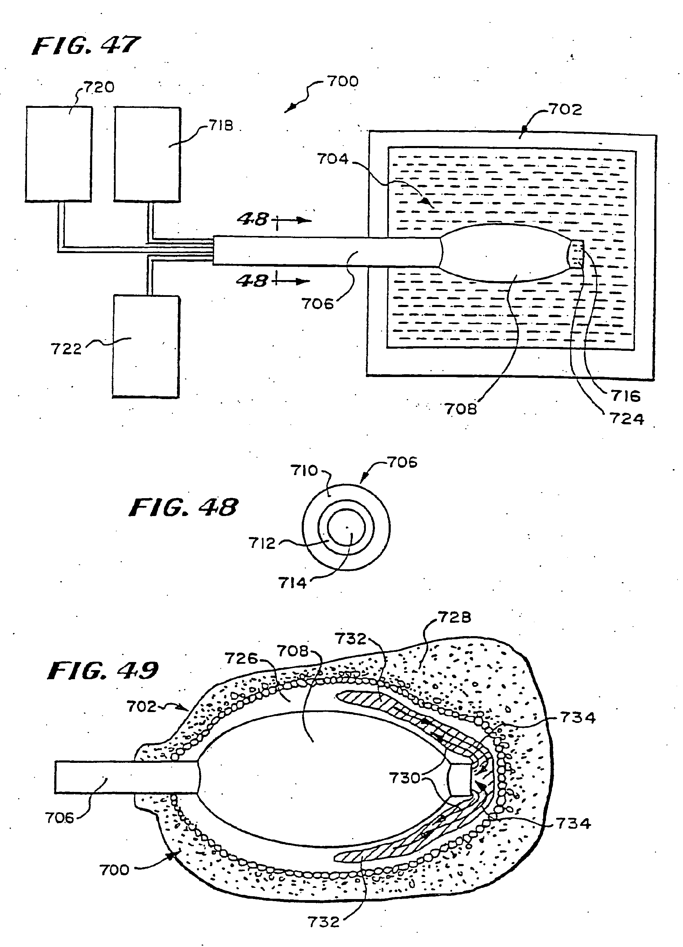

Fig. 47 shows a system 700 for harvesting bone marrow in a bone-marrow producing bone 702. The bone 702, which is shown diagrammatically in Fig. 47, can comprise, for example, the pelvis, or a vertebral body, or a distal radius.

The system 700 employs a bone marrow harvesting tool 704. The tool 704 includes a catheter tube 706, which carries an expandable body 708 at its distal end. The tool 704 can be deployed into the bone 702 using a minimally invasive approach, as previously described.

The catheter tube 706 has three concentric and independent lumens 710, 712, and 714 (see Fig. 48). The outer lumen 710 communicates with the interior of the body 78 and is coupled to a source 718 of an inflation liquid. The middle lumen 712 communicates with a source 720 of rinse liquid and a distal opening 716 on the catheter tube 706. The center lumen 714 communicates with a collection container 722 and a second distal opening 724 on the catheter tube 706.

The body 708 is deployed in a substantially collapsed condition, as already described. Inflation liquid, which is preferably radiopaque, is convey from the source 718 into the body 708 to expand it.

As Fig. 48 shows, the body 708 is constrained by selection of relatively inelastic materials or by exterior restraints (as previously described) to assume an elongated shape. Expansion of the body 708 creates a relatively shallow area of compaction 726 in cancellous bone 728 along a relatively long length. The size and shape of the body 708 will depend upon the geometry of the harvest site and the amount of bone marrow required. In long bones, like the distal radius, and in bones with narrow width but large area, such as the ribs or pelvis, the body 728 is shaped to compress a large area but not a great depth of cancellous bone 728.

As Fig. 48 also shows, as the body 708 expands, rinse liquid, which can be saline or another suitable biocompatible liquid, is conveyed from the source 720 into the area 726 (shown by arrows 730 in Fig. 48). The rinse liquid loosens up biological components (such as red blood cells, bone cells, and immune-β cells) within the defined area 726, forming component-rich suspension 732.

The body 708 is collapsed, and suction is applied through the lumen 714. The suction draws the component-rich suspension 732 from the area 726 into the collection container 722.

The above sequence of expansion, rinsing, collapse, and aspiration can be repeated to collect additional component-rich suspension 732 in the container 722. The position of the expandable body 708 in the bone 702 can be changed, if desired, to maintain a component-rich suspension 732 for harvesting.

Use of the expandable body 708 to form the long but shallow compaction area 726 permits the harvest of a significant concentration of therapeutic biological components with less damage to bone than conventional harvesting methods. If desired, standard casts or other fixation devices can be applied to the bone 702 after bone marrow harvesting until the bone 702 heals.

The features of the invention are set forth, in the following claims.

**Claims**

1. A system for treating a vertebral body having cortical bone surrounding an interior volume occupied, at least in part, by cancellous bone (32) comprising:

   a first outer guide sheath (72A) for providing a first access path into the interior volume of the vertebral body,

a second outer guide sheath (72B) for providing a second access path into the interior volume of the same vertebral body, the second access path being different than the first access path,
a first expandable body (56A) sized and configured to be introduced through one of the first and second guide sheaths into the interior volume to be enlarged to form a first void (84A) in the cancellous bone, and
a second expandable body (56B) sized and configured to be introduced through the other of the first and second guide sheaths into the interior volume to be enlarged to form a second void (84B) in the cancellous bone different than the first void.

2. A system according to claim 1 further including a filling tool having a nozzle, said filling tool being sized for passage into at least one of the first and second guide sheaths (72A, 72B) to deliver a filling material (96) into the respective first and/or second void.

3. A system according to claim 1 or 2, wherein the filling material (96) when hardened provides interior structural support for the cortical bone.

4. A system according to any one of claims 1 to 3, wherein each expandable body (56A, 56B) has a predetermined shape and size when expanded.

5. A system according to claim 4, wherein each expandable body (56A, 56B) includes a restraint that constrains expansion of the expandable body.

6. A system according to any one of claims 1 to 5, wherein the expandable bodies (56A, 56B) are inflatable.

7. A system according to any one of claims 1 to 6, wherein the expandable bodies (56A, 56B) when expanded compact cancellous bone.

8. A system according to any one of claims 1 to 7, wherein the first and second expandable bodies (56A, 56B) are carried by catheter tubes that are sized to pass through the respective first or second guide sheaths (72A, 72B).

**Patentansprüche**

1. System zum Behandeln eines Wirbelkörpers mit einer Knochenrinde (Kortikalis), die ein inneres Volumen umgibt, das zumindest teilweise von spongiösem Knochen (Spongiosa) (32) eingenommen wird, umfassend:

   eine erste äußere Führungshülse (72A) zum Bereitstellen eines ersten Zugangspfades in das innere Volumen des Wirbelkörpers,
   eine zweite äußere Führungshülse (72B) zum Bereitstellen eines zweiten Zugangspfades in das innere Volumen desselben Wirbelkörpers, wobei sich der zweite Zugangspfad von dem ersten Zugangspfad unterscheidet,
   einen ersten expandierbaren Körper (56A), der so groß und derart gestaltet ist, dass er durch eine von der ersten und zweiten Führungshülse in das innere Volumen eingeführt wird, das zu vergrößern ist, um einen ersten Hohlraum (84A) in dem spongiösen Knochen zu bilden, und
   einen zweiten expandierbaren Körper (56B), der so groß und derart gestaltet ist, dass er durch die andere von der ersten und zweiten Führungshülse in das innere Volumen eingeführt wird, das zu vergrößern ist, um einen zweiten Hohlraum (84B) in dem spongiösen Knochen zu bilden, der sich von dem ersten Hohlraum unterscheidet.

2. System nach Anspruch 1, des Weiteren enthaltend ein Füllwerkzeug mit einer Düse, wobei das Füllwerkzeug so groß ist, dass es in mindestens eine von der ersten und zweiten Führungshülse (72A, 72B) geht, um ein Füllmaterial (96) in den jeweils ersten und/oder zweiten Hohlraum abzugeben.

3. System nach Anspruch 1 oder 2, wobei das Füllmaterial (96), wenn es gehärtet ist, eine innere strukturelle Stütze für die Knochenrinde bereitstellt.

4. System nach einem der Ansprüche 1 bis 3, wobei jeder expandierbare Körper (56A, 56B), wenn er expandiert ist, eine vorbestimmte Form und Größe aufweist.

5. System nach Anspruch 4, wobei jeder expandierbare Körper (56A, 56B) eine Beschränkung aufweist, die die Expansion des expandierbaren Körpers beschränkt.

**6.** System nach einem der Ansprüche 1 bis 5, wobei die expandierbaren Körper (56A, 56B) aufblasbar sind.

**7.** System nach einem der Ansprüche 1 bis 6, wobei die expandierbaren Körper (56A, 56B), wenn sie expandiert sind, spongiösen Knochen verdichten.

**8.** System nach einem der Ansprüche 1 bis 7, wobei die ersten und zweiten expandierbaren Körper (56A, 56B) von Katheterschläuchen getragen werden, die so groß sind, dass sie durch die erste beziehungsweise zweite Führungshülse (72A, 72B) gehen.

**Revendications**

**1.** Système pour traiter un corps vertébral ayant un os cortical entourant un volume intérieur occupé, au moins en partie, par de l'os spongieux (32) comprenant :

un premier fourreau de guidage externe (72A) pour fournir un premier chemin d'accès dans le volume intérieur du corps vertébral,
un second fourreau de guidage externe (72B) pour fournir un second chemin d'accès dans le volume intérieur du même corps vertébral, le second chemin d'accès étant différent du premier chemin d'accès,
un premier corps dilatable (56A) dimensionné et configuré pour être introduit à travers l'un des premier et second fourreaux de guidage dans le volume intérieur à agrandir pour former un premier vide (84A) dans l'os spongieux, et
un second corps dilatable (56B) dimensionné et configuré pour être introduit à travers l'autre des premier et second fourreaux de guidage dans le volume intérieur à agrandir pour former un second vide (84B) dans l'os spongieux différent du premier vide.

**2.** Système selon la revendication 1, comprenant en outre un outil de remplissage ayant une buse, ledit outil de remplissage étant dimensionné pour un passage dans au moins l'un des premier et second fourreaux de guidage (72A, 72B) pour délivrer un matériau de remplissage (96) dans les premier et/ou second vides respectifs.

**3.** Système selon la revendication 1 ou 2, dans lequel le matériau de remplissage (96), lorsqu'il est durci, forme un support structurel intérieur pour l'os cortical.

**4.** Système selon l'une quelconque des revendications 1 à 3, dans lequel chaque corps dilatable (56A, 56B) a une forme et une taille prédéterminées lorsqu'il est dilaté.

**5.** Système selon la revendication 4, dans lequel chaque corps dilatable (56A, 56B) inclut un bridage qui contient la dilatation du corps dilatable.

**6.** Système selon l'une quelconque des revendications 1 à 5, dans lequel les corps dilatables (56A, 56B) sont gonflables.

**7.** Système selon l'une quelconque des revendications 1 à 6, dans lequel les corps dilatables (56A, 56B), lorsqu'ils sont dilatés, condensent le corps spongieux.

**8.** Système selon l'une quelconque des revendications 1 à 7, dans lequel les premier et second corps dilatables (56A, 56B) sont portés par des tubes de cathéter qui sont dimensionnés pour passer à travers le premier ou second fourreau de guidage respectif (72A, 72B).

**FIG. 1**

**FIG. 2**

**FIG. 3**

FIG. 4

FIG. 5A

FIG. 5B

EP 1 938 765 B1

FIG. 5C

FIG. 5D

FIG. 5E

*FIG. 5F*

*FIG. 5G*

*FIG. 5H*

EP 1 938 765 B1

FIG.5

FIG.5J(1)

FIG.5J(2)

FIG.5K(1)

FIG.5K(2)

EP 1 938 765 B1

FIG. 5L

FIG. 5M

FIG. 5N

**FIG. 50**

**FIG. 5P**

**FIG. 5Q**

EP 1 938 765 B1

FIG. 6

FIG. 7

EP 1 938 765 B1

FIG.8B

FIG.8C

FIG.8A

*FIG. 9*

*FIG. 10*

*FIG. 11*

EP 1 938 765 B1

## FIG. 12

DIRECTIONS

—79

## FIG. 13

EP 1 938 765 B1

FIG.15

FIG.14

FIG.17

FIG.18

FIG.16

# FIG.19

# FIG.21

# FIG.20

FIG. 22

309 379 317
310
315
312
314
377

FIG. 23

230
232
238
234

FIG. 24

25
241
246
240
246
242
25

## FIG. 25

## FIG. 26

# FIG. 27

# FIG. 28

FIG. 29A

## FIG.29B

FIG. 30

FIG. 30B

FIG. 31

FIG. 30C

FIG.33

FIG.32

**FIG.34**

304

302

300

309

307

306

**FIG.35**

300

300b

FIG.36A

**FIG.36B**

*FIG. 37A*

*FIG. 37B*

*FIG. 38*

*FIG.39B*

*FIG.39D*

*FIG.39A*

*FIG.39C*

*FIG. 41*

*FIG. 40*

# FIG. 42A

# FIG. 42B

# FIG. 42C

EP 1 938 765 B1

## FIG. 43

FIG. 44

622
616
624
614
626
628

632
618(2)
630
618(1)
620

FIG. 45

632
618(2)
630
618(1)

614

620

FIG. 46

622

616
614

618(2)
630

618(1)

*FIG. 47*

*FIG. 48*

*FIG. 49*

848

852    851    850    858    854

858    856    FIG. 50

864
858    860    858    854

850    856    862    FIG. 51

826  830    MR  842    FIG. 52
850
860
843
828    844
866    856
826    FIG. 53
860  869    882

MR
866
830
860
844
856
850
828
867    842
843

FIG. 54

FIG. 55

FIG. 56A

FIG. 56B

FIG. 57

FIG. 58A

FIG. 58B

FIG. 59

FIG. 60

FIG. 61

FIG. 62

FIG. 63

FIG. 64

FIG. 65

FIG. 66

FIG. 67

FIG. 68

1608    1606    1604

1602    1612

1600

1610

FIG. 69

1604    1614    1610

1608  1600

1606

1612

1616

FIG. 70

FIG. 71

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4969888 A
- US 5108404 A
- EP 0621020 A
- US 18822494 A
- US 48539495 A
- US 65967896 A